# EUROPEAN PATENT APPLICATION

(11) **EP 1 921 134 A1**
(43) Date of publication of application: **14.05.2008**
(21) Application number: 06123710.3
(22) Date of filing: 08.11.2006
(51) Int. Cl.: C12N 9/02, C12P 7/64

(54) **Method of producing fatty acid hydroperoxides**

(71) Applicant: Georg-August-Universität Göttingen, 37073 Göttingen (DE)
(72) Inventor: Feussner, Ivo, 37085, Göttingen (DE); Lang, Imke, 37085, Göttingen (DE)
(74) Representative: Neuefeind, Regina

(57) **Abstract**

The present invention relates to methods for producing fatty acid hydroperoxides. In particular, this invention relates to the production of C18 hydroperoxy polyunsaturated fatty acids, catalyzed by a specific 13-lipoxygenase (LOX) originating from a cyanobacterium. Further, the invention relates to methods of producing flavor ingredients and/or fragrance ingredients using the 13-LOX derived hydroperoxy fatty acids as starting compounds. The inventive 13-LOX is useful for the production of hydroperoxy fatty acids at a large-scale, and their further processing into flavor and fragrance ingredients.

SEQUENCE LISTING

<110>  Universität Göttingen

<120>  Method of producing fatty acid hydroperoxides

<130>  U 7082

<140>  EP 06 123 710.3
<141>  2006-11-08

<160>  16

<170>  PatentIn version 3.3

<210>  1
<211>  630
<212>  PRT
<213>  Nostoc punctiforme

<400>  1

Met Thr Ala Leu Ser Pro Asp His Ser Ile Ser Ser Ser Ser Thr His
1               5                   10                  15

Thr Leu Asp Ile Ala Arg Gln Glu Tyr Gln Tyr Asn Tyr Asn His Ile
            20                  25                  30

Pro Ser Ile Ala Met Val Asp Arg Leu Ser Ile Thr Glu Glu Phe Thr
        35                  40                  45

Thr Asn Trp Tyr Phe Leu Leu Ala Gln Gln Leu Arg Val Leu Phe Ile
    50                  55                  60

Asn Thr Leu Ile Val Asn Arg Gly Asn Gln Asp Ser Lys Ser Ile Arg
65                  70                  75                  80

Asp Asp Val Glu Arg Phe Ile Leu Glu Ala Leu Leu Lys Gly Ala Val
                85                  90                  95

Pro Ala Arg Ile Ser Ile Leu Ala Arg Ile Leu Gln Ile Ile Pro Gln
            100                 105                 110

Leu Leu Leu Lys Glu Ile Ser Lys Asp Phe Arg Glu Leu Asp Asp Leu
        115                 120                 125

Phe His Ser Ile Leu Lys Glu Asn Gly Leu Ala Ile Leu Arg Asp Ala
    130                 135                 140

Leu Lys Arg Ile Ile Thr Leu Leu Tyr Glu Gly Gln Pro Thr Gly His
145                 150                 155                 160

Ala Thr Ser Leu Lys Asp Tyr Glu Asn Leu Phe Pro Val Ile Ser Leu
                165                 170                 175

Pro Ala Ile Ala Lys Thr Tyr Gln Glu Asp Glu Val Phe Ala Tyr Met
            180                 185                 190

Arg Val Ala Gly Tyr Asn Pro Val Thr Ile Lys Arg Val Thr Thr Leu
        195                 200                 205

Ser Asp Arg Phe Pro Val Thr Asp Glu His Tyr Gln Ala Val Met Gly
    210                 215                 220

Thr Asp Asp Ser Leu Ala Ala Ala Gly Ile Glu Gly Arg Leu Tyr Leu
225                 230                 235                 240

Ala Asp Tyr Lys Ile Leu Asp Gly Ala Ile Asn Gly Thr Phe Pro His
                245                 250                 255

Glu Gln Lys Tyr Leu Tyr Ala Pro Leu Ala Leu Phe Ala Leu Pro Lys
            260                 265                 270

Gly Ser Asp Pro Thr Arg Leu Leu Arg Pro Val Ala Ile Gln Cys Gly
        275                 280                 285

Gln Thr Pro Gly Pro Asp Tyr Pro Ile Val Thr Pro Asn Ser Gly Lys
    290                 295                 300

Tyr Ala Trp Leu Phe Ala Lys Thr Ile Val Gln Ile Ala Asp Ala Asn
305                 310                 315                 320

Ile His Glu Ala Val Thr His Leu Ala Arg Thr His Leu Leu Val Gly
                325                 330                 335

Val Phe Ala Ile Ala Thr Ala Arg Gln Leu Pro Leu Thr His Pro Leu
            340                 345                 350

Arg Ile Leu Leu Arg Pro His Phe Asp Ser Thr Leu Ala Ile Asn Asp
        355                 360                 365

Ala Ala Gln Arg Ile Leu Ile Ala Pro Gly Gly Gly Val Asp Arg Leu
    370                 375                 380

Leu Ser Ser Ser Ile Asp Asn Ser Arg Val Leu Ala Val Leu Gly Leu
385                 390                 395                 400

Gln Ser Tyr Ser Phe Asn Ser Thr Ile Leu Pro Asn Gln Phe Gln Gln
                405                 410                 415

Arg Gly Val Asp Asp Pro Asn Leu Leu Pro Ile Tyr Pro Tyr Arg Asp
            420                 425                 430

Asp Ala Leu Leu Ile Trp Asn Ala Ile His Gln Trp Val Trp Asp Tyr
        435                 440                 445

Leu Asn Leu Tyr Tyr Thr Thr Asp Glu Asp Ile Gln Lys Asp Arg Ala
    450                 455                 460

Leu Gln Ala Trp Ala Ala Glu Ile Pro Ala Tyr Asp Gly Gly Arg Ile
465                 470                 475                 480

Pro Asp Phe Gly Glu Asp Gly Gly Ile Lys Thr Leu Asn Tyr Leu Ile
                485                 490                 495

Asp Ala Thr Thr Leu Ile Ile Phe Thr Ala Ser Ala Gln His Ala Ala
            500                 505                 510

Val Asn Phe Pro Gln Lys Asp Leu Met Gly Tyr Ala Ala Ala Ile Pro
        515                 520                 525

Leu Ala Gly Tyr Leu Pro Ala Ser Thr Leu Lys Arg Glu Val Thr Glu
    530                 535                 540

Gln Asp Tyr Leu Asn Leu Leu Pro Pro Leu Asp Gln Ala Gln Arg Gln
545                 550                 555                 560

Tyr Asn Leu Leu Ser Leu Leu Gly Ser Val Tyr Tyr Asn Lys Leu Gly
                565                 570                 575

Glu Tyr Glu Gln Gly Tyr Phe Thr Asp Glu Lys Val Lys Pro Leu Leu
            580                 585                 590

Gln Ala Phe Gln Ser His Leu Gln Gln Val Glu Asn Thr Ile Lys Gln
        595                 600                 605

Arg Asn Leu His Arg Pro Pro Tyr Glu Tyr Leu Leu Pro Ser Lys Ile
    610                 615                 620

Pro Gln Ser Ile Asn Ile
625                 630

<210>  2
<211>  547
<212>  PRT
<213>  Nostoc punctiforme

<400>  2

Met Lys Pro Tyr Leu Pro Gln Asn Asp Pro Asp Pro Thr Lys Arg Gln
1               5                   10                  15

Ile Leu Leu Glu Arg Asn Gln Gly Glu Tyr Glu Phe Asp Tyr Asp Phe
            20                  25                  30

Leu Val Pro Met Ala Met Leu Lys Asn Val Pro Ser Ile Glu Asn Phe
        35                  40                  45

Ser Thr Lys Tyr Ile Ala Glu Arg Thr Leu Glu Thr Ala Glu Leu Pro
    50                  55                  60

Ile Asn Met Leu Ala Val Lys Thr Arg Ser Leu Trp Asp Pro Leu Asp
65                  70                  75                  80

Glu Leu Gln Asp Tyr Glu Asp Tyr Phe Pro Val Leu Pro Lys Pro Asn
                85                  90                  95

Ile Ile Lys Thr Tyr Gln Ser Asp Asp Ser Phe Cys Glu Gln Arg Leu
            100                 105                 110

Cys Gly Ala Asn Pro Phe Val Leu Arg Arg Ile Glu Gln Met Pro Asp
        115                 120                 125

Gly Phe Ala Phe Thr Ile Leu Glu Leu Gln Glu Lys Phe Gly Asp Ser
    130                 135                 140

Ile Asn Leu Val Glu Lys Leu Ala Asn Gly Asn Leu Tyr Val Ala Asp
145                 150                 155                 160

Tyr Arg Ala Leu Ala Phe Val Lys Gly Gly Ser Tyr Glu Arg Gly Lys
                165                 170                 175

Lys Phe Leu Pro Thr Pro Ile Ala Phe Phe Cys Trp Arg Ser Ser Gly
            180                 185                 190

Phe Ser Asp Arg Gly Gln Leu Val Pro Ile Val Ile Gln Ile Asn Pro
        195                 200                 205

Ala Asp Gly Lys Gln Ser Gln Leu Ile Thr Pro Phe Asp Asp Pro Leu
    210                 215                 220

Thr Trp Phe His Ala Lys Leu Cys Val Gln Ile Ala Asp Ala Asn His
225                 230                 235                 240

His Glu Met Ser Ser His Leu Cys Arg Thr His Phe Val Met Glu Pro
                245                 250                 255

Phe Ala Ile Val Thr Ala Arg Gln Leu Ala Glu Asn His Pro Leu Ser
            260                 265                 270

Leu Leu Leu Lys Pro His Phe Arg Phe Met Leu Ala Asn Asn Asp Leu
        275                 280                 285

Ala Arg Lys Arg Leu Ile Ser Arg Gly Gly Pro Val Asp Glu Leu Leu
    290                 295                 300

Ala Gly Thr Leu Gln Glu Ser Leu Gln Ile Val Val Asn Ala Tyr Thr
305                 310                 315                 320

Glu Trp Ser Leu Asp Gln Phe Ser Leu Pro Thr Glu Leu Lys Asn Arg
                325                 330                 335

Gly Met Asp Asp Pro Asp Asn Leu Pro His Tyr Pro Tyr Arg Asp Asp
            340                 345                 350

Gly Leu Leu Leu Trp Asn Ala Ile Lys Lys Phe Val Ser Glu Tyr Leu
        355                 360                 365

Gln Ile Tyr Tyr Lys Thr Pro Gln Asp Leu Ala Glu Asp Leu Glu Leu
    370                 375                 380

Gln Ser Trp Val Gln Glu Leu Val Ser Gln Ser Gly Gly Arg Val Lys
385                 390                 395                 400

Gly Ile Ser Asp Arg Ile Asn Thr Leu Asp Gln Leu Val Asp Ile Ala
                405                 410                 415

Thr Ala Val Ile Phe Thr Cys Gly Pro Gln His Ala Ala Val Asn Tyr
            420                 425                 430

Ser Gln Tyr Glu Tyr Met Thr Phe Met Pro Asn Met Pro Leu Ala Ala
        435                 440                 445

Tyr Lys Gln Met Thr Ser Glu Gly Thr Ile Pro Asp Arg Lys Ser Leu
    450                 455                 460

Leu Ser Phe Leu Pro Pro Ser Lys Gln Thr Ala Asp Gln Leu Ser Ile
465                 470                 475                 480

Leu Phe Ile Leu Ser Ala Tyr Arg Tyr Asp Arg Leu Gly Tyr Tyr Asp
                485                 490                 495

Asp Lys Phe Leu Asp Pro Glu Ala Gln Asp Val Leu Ala Lys Phe Gln
            500                 505                 510

Gln Glu Leu Asn Glu Ala Glu Arg Glu Ile Glu Leu Asn Asn Lys Ser
        515                 520                 525

Arg Leu Ile Asn Tyr Asn Tyr Leu Lys Pro Arg Leu Val Thr Asn Ser
    530                 535                 540

Ile Ser Val
545

<210>  3
<211>  1893
<212>  DNA
<213>  Nostoc punctiforme

<400>  3
atgactgctt tatcaccaga tcattcaatc agttcatcaa gtactcatac tttagatata      60

gctaggcaag agtatcaata taactacaac catattccat ctattgcgat ggtggatcgg     120

ctttctatta cggaagagtt caccactaac tggtattttt tattagccca gcagttacga     180

gttcttttta tcaatacctt gattgtcaac agaggcaatc aagattctaa atcgattcgt 240
gatgatgtcg aaagatttat tttagaagcc ttgctcaagg gagcagtacc agcccgaatc 300
agcattctgg caagaatcct gcaaattatc cctcagcttt tgctcaaaga gatatctaag 360
gattttagag aactcgacga tctttttcat tctattctga aagaaaacgg acttgcgatc 420
ctcagagatg ctctaaagag gataattacc cttctatatg aaggacagcc tacaggacat 480
gcaaccagtc tcaaggacta cgaaaatttg tttccggtga ttagtctacc agccatcgct 540
aaaacttatc aggaagatga agtatttgcc tacatgagag tagctggcta caatcccgtc 600
acgatcaagc gagtaactac tctaagcgat cgcttcccag tcacagacga acattaccaa 660
gcggtgatgg gaactgacga ttcattagca gcagcaggga ttgaaggcag gctctactta 720
gctgactata aaattttaga tggtgcaatc aacggtacat tcccacacga gcaaaaatat 780
ctctatgctc ccctagcact atttgcctta cccaaaggtt cagatcccac ccgtctattg 840
cgtccggtag ccattcaatg cggtcagacc ccaggcccag attatccaat tgttactcct 900
aactccggta aatacgcttg gcttttgccc aaaacgattg tccaaatagc agatgctaac 960
atccacgaag ctgttactca cctagcaaga actcatcttt tagttggtgt ttttgcgatc 1020
gcaactgctc gacaattgcc actcacacat cccctcagaa tcctgctgcg cccgcatttt 1080
gatagcacct tagcaattaa cgatgccgcc caacggattc tcattgctcc aggcggtggt 1140
gtcgatagat tactctcatc atcaatcgat aactctcgcg ttttagcagt gctagggttg 1200
caaagctata gttttaacag caccatctta cctaatcaat tccaacagcg cggtgtagac 1260
gatcctaatc tattgcctat ttatccttac cgggatgatg ccctactaat ctggaatgcc 1320
attcatcaat gggtttggga ctacctgaac ctttactaca ccacggatga agacattcaa 1380
aaagacagag cattgcaagc gtgggcagcc gaaatcccag cttacgatgg tggtcgtatc 1440
cccgattttg gcgaagatgg gggcatcaaa acgctaaatt acctgattga tgccactaca 1500
ttaattattt ttactgccag tgcccaacat gcagccgtta actttccgca aaaagatttg 1560
atgggctacg ccgcagccat accattggca ggttatttac cagcctcaac tctcaaaaga 1620
gaagttaccg agcaagacta ccttaatctg ctcccccctt tagatcaggc acaacggcaa 1680
tacaacctac tcagcttatt agggtctgta tactacaaca agctgggcga gtatgagcaa 1740
ggatacttca cagacgagaa agtaaaacca ttgttacagg cattccaaag tcatcttcag 1800
caggtagaaa ataccatcaa gcagcgtaat ttgcaccgcc caccctatga gtatctcctt 1860
ccttctaaaa ttcctcagag catcaatatc tga 1893

<210> 4
<211> 1644
<212> DNA
<213> Nostoc punctiforme

<400> 4
atgaaaccat acctccctca gaatgatcct gaccctacaa aacgtcaaat attgctagag 60

agaaatcaag gggagtatga atttgattac gactttttgg tacctatggc aatgctaaaa 120
aatgtacctt ctatagaaaa cttttcaact aagtatattg ctgaacggac attagagaca 180
gcagaactgc ctataaatat gttagccgtt aaaacccgtt ctttatggga ccctttagat 240
gaattgcaag actatgaaga ctattttcca gttttgccta aacctaatat tatcaaaaca 300
taccaaagtg atgactcttt ttgtgagcaa cggctttgtg gggcaaatcc ttttgtttta 360
cgtcgaattg agcagatgcc agatggcttc gcctttacca ttttagaatt gcaagaaaaa 420
tttggtgact ctattaactt agtagaaaaa cttgcgaatg gaaatttata tgtagctgat 480
tacagagcgc ttgcgtttgt taaaggaggt agttatgaaa gaggtaagaa gtttttacca 540
acccctatag ctttcttttg ttggcgcagt tctggtttta gcgatcgcgg tcaactagta 600
ccgattgtta tccaaatcaa ccccgcagat ggcaaacaga gccagctaat tacacctttc 660
gatgaccctt taacctggtt tcatgccaag ctttgtgttc aaattgctga tgctaaccat 720
catgaaatga gtagccatct gtgtcgaact cactttgtta tggaaccctt tgctattgtc 780
acagcccgtc aactagccga gaaccatccc cttagcttac tgctaaaacc ccacttccgt 840
ttcatgttgg ctaataatga cttggctcgt aagcgcctaa ttagtagagg tgggcctgtt 900
gacgaattgc tagccggaac tctgcaagag tcattgcaaa ttgtcgttaa cgcatataca 960
gaatggagct tagatcagtt ttccttacct actgaactaa aaaatcgggg tatggatgat 1020
ccagacaact tacctcacta tccctatcga gacgatggct tattattgtg gaatgccatt 1080
aaaaagtttg tgtctgaata cttgcagata tactacaaaa ctccccaaga tttagcagaa 1140
gacttggaat tacaaagttg ggtgcaggaa ttagtttccc aatcaggcgg acgagtcaag 1200
ggtattagcg accgcatcaa cacattagac caattagttg atattgctac tgcggttatc 1260
ttcacctgtg ggccgcaaca cgctgctgtt aactactcac aatatgaata tatgactttc 1320
atgccaaata tgcctcttgc tgcttataaa caaatgacat cagaaggcac tattcctgac 1380
cgtaaaagtc tattatcatt tctgccaccg tcaaagcaaa ctgctgacca attatcgatt 1440
ttatttatcc tgtcggccta ccgttatgac agattagggt actacgatga taaattttta 1500
gacccagagg ctcaggatgt tttagctaaa ttccagcagg agttgaatga agcagagcgg 1560
gaaattgagt tgaataacaa gagtcgttta ataaattaca actatctcaa accaaggctt 1620
gtgactaata gtattagcgt gtaa 1644

<210> 5
<211> 36
<212> DNA
<213> Artificial

<220>
<223> synthetic DNA primer

<400> 5
atgactgctt tatcaccaga tcattcaatc agttca 36

<210> 6

<211>  28
<212>  DNA
<213>  Artificial

<220>
<223>  synthetic DNA primer

<400>  6
tcagatattg atgctctgag gaatttta                                          28


<210>  7
<211>  27
<212>  DNA
<213>  Artificial

<220>
<223>  synthetic DNA primer

<400>  7
atgaaaccat acctccctca gaatgat                                           27


<210>  8
<211>  27
<212>  DNA
<213>  Artificial

<220>
<223>  synthetic DNA primer

<400>  8
tcacacgcta atactatttg acacaag                                           27


<210>  9
<211>  34
<212>  DNA
<213>  Artificial

<220>
<223>  synthetic DNA primer

<400>  9
cgcggatcca tgactgcttt atcaccagat catt                                   34


<210>  10
<211>  34
<212>  DNA
<213>  Artificial

<220>
<223>  synthetic DNA primer

<400>  10
tgcactgcag tcagatattg atgctctgag gaat                                   34


<210>  11
<211>  32
<212>  DNA
<213>  Artificial

<220>
<223>  synthetic DNA primer

<400>  11

cggatccatg aaaccatacc tccctcagaa tg                                    32

<210>  12
<211>  34
<212>  DNA
<213>  Artificial

<220>
<223>  synthetic DNA primer

<400>  12
gctgcagtta cacgctaata ctattagtca caag                                  34

<210>  13
<211>  685
<212>  PRT
<213>  Pseudomonas aeruginosa

<300>
<308>  UniProtKB TrEMBL/AAL85880
<309>  2002-06-01
<313>  (1)..(685)

<400>  13

Met Lys Arg Arg Ser Val Leu Leu Ser Gly Val Ala Leu Ser Gly Thr
1               5                   10                  15

Ala Leu Ala Asn Asp Ser Ile Phe Phe Ser Pro Leu Lys Tyr Leu Gly
            20                  25                  30

Ala Glu Gln Gln Arg Ser Ile Asp Ala Ser Arg Ser Leu Leu Asp Asn
        35                  40                  45

Leu Ile Pro Pro Ser Leu Pro Gln Tyr Asp Asn Leu Ala Gly Lys Leu
    50                  55                  60

Ala Arg Arg Ala Val Leu Thr Ser Lys Lys Leu Ala Tyr Val Trp Thr
65                  70                  75                  80

Glu Asn Phe Ala Asn Val Lys Gly Val Pro Met Ala Arg Ser Val Pro
                85                  90                  95

Leu Gly Glu Leu Pro Asn Val Asp Trp Leu Leu Lys Thr Ala Gly Val
            100                 105                 110

Ile Val Glu Leu Ile Val Asn Ser Phe Ala Ser Leu Pro Ala Ser Ala
        115                 120                 125

Ala Ala Gln Phe Glu Arg Ile Pro Ala Gly Leu Asn Gly Asp Leu Glu
    130                 135                 140

Ala Ala Arg Gln Val His Glu Ala Leu Leu Glu Glu Ala Lys Asn Asp
145                 150                 155                 160

Pro Ala Ala Ala Gly Ser Leu Leu Leu Arg Phe Thr Glu Leu Gln Thr

                165                 170                 175

Arg Val Ile Ala Leu Leu Thr Arg Val Gly Leu Leu Val Asp Asp Ile
            180                 185                 190

Leu Lys Ser Ala Ser Asn Leu Val Thr Gln Gly Gly Gln Gly Asp Gly
        195                 200                 205

Leu Asn Arg Phe Arg Ala Val Phe Gly Thr Leu Arg Leu Pro Glu Val
    210                 215                 220

Ala Asp Ser Phe Arg Asp Asp Glu Ala Phe Ala Tyr Trp Arg Val Ala
225                 230                 235                 240

Gly Pro Asn Pro Leu Leu Ile Arg Arg Val Asp Ala Leu Pro Ala Asn
                245                 250                 255

Phe Pro Leu Gly Glu Glu Gln Phe Arg Arg Val Met Gly Ala Asp Asp
            260                 265                 270

Ser Leu Leu Glu Ala Ala Ala Ser Arg Arg Leu Tyr Leu Leu Asp Tyr
        275                 280                 285

Ala Glu Leu Gly Lys Leu Ala Pro Ser Gly Ala Val Asp Lys Leu Leu
    290                 295                 300

Thr Gly Thr Gly Phe Ala Tyr Ala Pro Ile Ala Leu Phe Ala Leu Gly
305                 310                 315                 320

Lys Asp Arg Ala Arg Leu Leu Pro Val Ala Ile Gln Cys Gly Gln Asp
                325                 330                 335

Pro Ala Thr His Pro Met Phe Val Arg Pro Ala Glu Ser Glu Ser Asp
            340                 345                 350

Leu Tyr Trp Gly Trp Gln Met Ala Lys Thr Val Val Leu Val Ala Glu
        355                 360                 365

Glu Asn Tyr His Glu Met Phe Val His Leu Ala Gln Thr His Leu Val
    370                 375                 380

Ser Glu Ala Phe Cys Leu Ala Thr Gln Arg Thr Leu Ala Pro Ser His
385                 390                 395                 400

Pro Leu His Val Leu Leu Ala Pro His Phe Glu Gly Thr Leu Phe Val
                405                 410                 415

Asn Glu Gly Ala Ala Arg Ile Leu Leu Pro Ser Ala Gly Phe Ile Asp
            420                 425                 430

Val Met Phe Ala Ala Pro Ile Gln Asp Thr Gln Ala Thr Ala Gly Gly

        435                 440                 445

Asn Arg Leu Gly Phe Asp Phe Tyr Arg Gly Met Leu Pro Glu Ser Leu
    450                 455                 460

Lys Ala Arg Asn Val Asp Asp Pro Ala Ala Leu Pro Asp Tyr Pro Tyr
465                 470                 475                 480

Arg Asp Asp Gly Leu Leu Val Trp Asn Ala Ile Arg Gln Trp Ala Ala
                485                 490                 495

Asp Tyr Val Ala Val Tyr Tyr Ala Ser Asp Gly Asp Val Thr Ala Asp
            500                 505                 510

Val Glu Leu Ala Ala Trp Val Gly Glu Val Ile Gly Ser Gly Lys Val
        515                 520                 525

Ala Gly Phe Arg Pro Ile Thr Gly Arg Ser Gln Leu Val Glu Val Leu
    530                 535                 540

Thr Met Val Ile Phe Thr Ala Ser Ala Gln His Ala Ala Val Asn Phe
545                 550                 555                 560

Pro Gln Pro Ser Met Met Thr Tyr Ala Pro Ala Ile Cys Ala Met Ser
                565                 570                 575

Ala Ala Pro Ala Pro Asp Ser Pro Ser Gly Lys Ser Glu Ala Asp Trp
            580                 585                 590

Leu Lys Met Met Pro Pro Ala Leu Val Ala Leu Glu Lys Val Asn Ile
        595                 600                 605

Tyr His Leu Leu Gly Ser Val Tyr His Gly Arg Leu Gly Asp Tyr Arg
    610                 615                 620

Gln Thr Gly Phe Pro Tyr Ala Pro Val Phe Ser Asp Arg Arg Val Thr
625                 630                 635                 640

Ala Ser Gly Gly Pro Leu Glu Arg Phe Gln Ala Arg Leu Lys Glu Val
                645                 650                 655

Glu Ala Thr Ile Arg Thr Arg Asn Gln Ala Arg Arg Lys Pro Tyr Glu
            660                 665                 670

Tyr Leu Leu Pro Ser Arg Ile Pro Ala Ser Thr Asn Ile
        675                 680                 685

<210> 14
<211> 919
<212> PRT
<213> Arabidopsis thaliana

<300>
<308>  TrEMBL/AAF79461
<309>  2000-10-01
<313>  (1)..(919)

<400>  14
   Met Ala Leu Ala Lys Glu Leu Met Gly Tyr Pro Leu Ile Thr Glu Arg
   1               5                   10                  15
   Ser Ser Leu Val Ser Ser Ala Ser His Phe Lys Lys Arg Thr Gln Ser
               20                  25                  30
   Thr Gln Phe Ser Ile Asn Pro Phe Asp Arg Arg Pro Arg Lys Thr Lys
           35                  40                  45
   Ser Gly Val Val Ala Ala Ile Ser Glu Asp Leu Val Lys Thr Leu Arg
       50                  55                  60
   Phe Ser Thr Thr Thr Gly Asp Arg Lys Ser Glu Glu Glu Glu Lys Ala
   65                  70                  75                  80
   Ala Val Lys Phe Lys Val Arg Ala Val Val Thr Val Arg Asn Lys Asn
                   85                  90                  95
   Lys Glu Asp Leu Lys Glu Thr Leu Val Lys His Leu Asp Ala Phe Ala
               100                 105                 110
   Asp Lys Ile Gly Arg Asn Ile Val Leu Glu Leu Ile Ser Thr Gln Leu
           115                 120                 125
   Asp Pro Lys Thr Lys Leu Pro Lys Lys Ser Asn Ala Ala Val Leu Lys
       130                 135                 140
   Asp Trp Ser Lys Lys Ser Lys Thr Lys Ala Glu Arg Val His Tyr Thr
   145                 150                 155                 160
   Ala Glu Phe Thr Val Asp Ala Ala Phe Gly Ser Pro Gly Ala Ile Thr
                   165                 170                 175
   Val Met Asn Lys His Gln Lys Glu Phe Phe Leu Glu Ser Ile Thr Ile
               180                 185                 190
   Glu Gly Phe Ala Leu Gly Pro Val His Phe Pro Cys Asn Ser Trp Val
           195                 200                 205
   Gln Ser Gln Lys Asp His Pro Asp Lys Arg Ile Phe Phe Thr Asn Gln
       210                 215                 220
   Pro Tyr Leu Pro Asn Glu Thr Pro Ser Gly Leu Arg Val Leu Arg Glu
   225                 230                 235                 240
   Lys Glu Leu Lys Asn Leu Arg Gly Asp Gly Ser Gly Val Arg Lys Leu
   245 250 255
   Ser Asp Arg Ile Tyr Asp Phe Asp Val Tyr Asn Asp Leu Gly Asn Pro
   260 265 270
   Asp Lys Ser Ser Glu Leu Ser Arg Pro Lys Leu Gly Gly Lys Glu Val
   275 280 285
   Pro Tyr Pro Arg Arg Cys Arg Thr Gly Arg Gln Ser Thr Val Ser Asp
   290 295 300
   Lys Asp Ala Glu Ser Arg Val Glu Lys Pro Leu Pro Met Tyr Val Pro
   305 310 315 320
   Arg Asp Glu Gln Phe Glu Glu Ser Lys Gln Asp Thr Phe Ala Ala Gly
   325 330 335
   Arg Leu Lys Ala Val Leu His His Leu Ile Pro Ser Leu Lys Ala Ser
   340 345 350
   Ile Val Ala Glu Asp Phe Ala Asp Phe Gly Glu Ile Asp Arg Leu Tyr
   355 360 365
   Lys Glu Gly Leu Leu Leu Lys Leu Gly Phe Gln Asp Asp Ile Phe Lys
   370 375 380
   Lys Phe Pro Leu Pro Lys Val Val Val Asp Thr Leu Gln Glu Ser Thr
   385 390 395 400
   Lys Gly Leu Leu Lys Tyr Asp Thr Pro Lys Ile Leu Ser Lys Asp Lys
   405 410 415
   Asn Ala Trp Leu Arg Asp Asp Glu Phe Ala Arg Gln Ala Ile Ala Gly
   420 425 430
   Ile Asn Pro Val Asn Ile Glu Arg Val Lys Thr Phe Pro Pro Val Ser
   435 440 445
   Asn Leu Asp Pro Lys Ile Tyr Gly Pro Gln His Ser Ala Leu Thr Asp
   450 455 460
   Asp His Ile Ile Gly His Leu Asp Gly Phe Ser Val Gln Gln Ala Leu
   465 470 475 480
   Glu Glu Asn Arg Leu Tyr Met Leu Asp Tyr His Asp Ile Phe Leu Pro
   485 490 495
   Phe Leu Asp Arg Ile Asn Ala Leu Asp Gly Arg Lys Ala Tyr Ala Thr
   500 505 510
   Arg Thr Ile Phe Phe Leu Thr Arg Leu Gly Thr Leu Lys Pro Val Ala
   515 520 525
   Ile Glu Leu Ser Leu Pro Pro His Gly Pro Lys His Arg Ser Lys Arg
   530 535 540
   Val Leu Thr Pro Pro Val Asp Ala Thr Ser Asn Trp Met Trp Gln Leu
   545 550 555 560
   Ala Lys Ala His Val Ser Ser Asn Asp Ala Gly Val His Gln Leu Val
   565 570 575
   Asn His Trp Leu Arg Thr His Ala Cys Leu Glu Pro Phe Ile Leu Ala
   580 585 590
   Ala His Arg Gln Leu Ser Ala Met His Pro Ile Phe Lys Leu Leu Asp
   595 600 605
   Pro His Met Arg Tyr Thr Leu Glu Ile Asn Ala Leu Ala Arg Gln Ser
   610 615 620
   Leu Ile Ser Ala Asp Gly Val Ile Glu Gly Gly Phe Thr Ala Gly Ala
   625 630 635 640
   Tyr Gly Met Glu Met Ser Ala Ala Ala Tyr Lys Ser Ser Trp Arg Phe
   645 650 655
   Asp Met Glu Gly Leu Pro Ala Asp Leu Ile Arg Arg Gly Met Ala Ile
   660 665 670
   Pro Asp Ala Thr Gln Pro His Gly Leu Lys Leu Leu Ile Glu Asp Tyr
   675 680 685
   Pro Tyr Ala Asn Asp Gly Leu Leu Leu Trp Ser Ala Ile Gln Thr Trp
   690 695 700
   Val Arg Thr Tyr Val Glu Arg Tyr Tyr Pro Asn Pro Asn Leu Ile Lys
   705 710 715 720
   Thr Asp Ser Glu Leu Gln Ser Trp Tyr Ser Glu Ser Ile Asn Val Gly
   725 730 735
   His Ala Asp Leu Arg Asp Ala Asp Trp Trp Pro Glu Leu Ser Thr Val
   740 745 750
   Asp Asp Leu Val Ser Ile Leu Thr Thr Leu Ile Trp Leu Ala Ser Ala
   755 760 765
   Gln His Ala Ala Leu Asn Phe Gly Gln Tyr Pro Tyr Gly Gly Tyr Val
   770 775 780
   Pro Asn Arg Pro Pro Leu Met Arg Arg Leu Ile Pro Asp Glu Ser Asp
   785                 790                 795                 800
   Pro Glu Tyr Ala Ser Phe Ile Ser His Pro Glu Lys Tyr Tyr Phe Ser
                   805                 810                 815
   Ser Met Pro Ser Leu Ala Gln Thr Ser Lys Phe Met Ala Val Val Asp
               820                 825                 830
   Thr Leu Ser Thr His Ser Pro Asp Glu Glu Tyr Ile Gly Glu Arg Gln
           835                 840                 845
   Gln Pro Ser Ile Trp Thr Gly Asp Ala Glu Ile Val Glu Ala Phe Tyr
       850                 855                 860
   Gly Phe Ala Ala Glu Ile Gly Arg Ile Glu Lys Glu Ile Glu Lys Arg
   865                 870                 875                 880
   Asn Ala Asp Pro Asp Arg Arg Asn Arg Cys Gly Ala Gly Val Leu Pro
                   885                 890                 895
   Tyr Glu Leu Leu Val Pro Ser Ser Glu Pro Gly Val Thr Cys Arg Gly
               900                 905                 910
   Val Pro Asn Ser Val Ser Ile
           915

<210>  15
<211>  937
<212>  PRT
<213>  Physcomitrella patens

<300>
<308>  TrEMBL/CAE47464
<309>  2004-07-05
<313>  (1)..(937)

<400>  15

Met Met Leu Asn Arg Leu Glu Thr Gly Ser Leu Ala Ser Ala Phe Arg
1               5                   10                  15

Gly Ser Arg Glu Glu Phe Val Val Ser Arg Gly Arg Ser Pro Gly Arg
            20                  25                  30

Ser Gln Ser Ile Gln Val Arg Ala Ser Ser Gly Gly Asp Pro Ala Gly
        35                  40                  45

Trp Leu Gln Thr Ala Ser Lys Gln Leu Gly Lys Leu Ser Ser Phe Gly
    50                  55                  60

Glu Lys Arg Lys Thr Ala Thr Ser Thr Ser Thr Arg Gly Pro Ser Gly
65                  70                  75                  80

Asp Asn Val Gln Tyr Thr Gly Val Ala Thr Thr Met Lys Lys Leu Lys

                85                  90                  95

Val Leu Asp Leu Ile Asp Arg Val Ala Asp Ile Gln Asp Asp Thr Ser
            100                 105                 110

Glu Ile Val Gly Gly Lys Arg Val Thr Val Gln Leu Val Ser Lys Asp
        115                 120                 125

Val Asp Pro Lys Thr Gly Glu Ser Met Lys Ser Ser Glu Val Ile Phe
    130                 135                 140

Pro Asn Trp Ala Gly Leu Glu Gly Pro Ala Ala Ser Leu Ile Asp Phe
145                 150                 155                 160

Val Leu Glu Phe Thr Val Pro Lys Ser Phe Gly Val Pro Gly Ala Ile
                165                 170                 175

Leu Val Lys Asn Ala His Pro Asn Glu Phe Leu Leu Val Ser Phe Glu
            180                 185                 190

Leu Glu Leu His Asp Lys Ser Lys Ala His Tyr Val Thr Asn Ser Trp
        195                 200                 205

Val Tyr Asn Thr Glu Lys Thr Gly Ala Arg Ile Phe Phe Gln Asn Thr
    210                 215                 220

Ala Tyr Leu Pro Asp Glu Thr Pro Ala Ser Leu Lys Ala Leu Arg Glu
225                 230                 235                 240

Gln Glu Leu Ile Asn Leu Arg Gly Asp Gly Thr Gly Glu Arg Gln Ile
                245                 250                 255

Gly Asp Arg Ile Tyr Asp Tyr Ala Val Tyr Asn Asp Leu Gly Asn Ile
            260                 265                 270

Glu Gln Asn Glu Lys Phe Glu Arg Pro Asn Leu Gly Gly Asn Asp Met
        275                 280                 285

Tyr His Phe Pro Arg Arg Met Arg Thr Gly Arg Arg Asn Thr Thr Val
    290                 295                 300

Glu Ala Lys Lys Phe Pro Gly Met Val Tyr Glu Thr Arg Lys Thr Lys
305                 310                 315                 320

Gly Asp Phe Tyr Ile Pro Arg Asp Glu Ala Phe Glu Arg Ala Lys Met
                325                 330                 335

Ser Asp Phe Leu Ala Asp Gly Phe Arg Ser Ile Gly His Ser Val Ser
            340                 345                 350

Ser Lys Val Thr Gly Phe Val Thr Arg Lys Gln Glu Phe Asp Thr Val

        355                 360                 365

Glu Glu Ile Lys Lys Leu Tyr Ala Lys Lys Gly Glu Lys Val Gly Gly
    370                 375                 380

Ile Asn Asn Val Leu Pro Asp Lys Glu Asp Ile Pro Glu Gln Glu Gln
385                 390                 395                 400

Tyr Pro Leu Val Phe Leu Gln Glu Val Leu Lys Pro Asp Gly Lys Met
                405                 410                 415

Glu His Pro Leu Leu Tyr Pro Leu Pro Gln Leu Leu Gln Ala Asp Asp
            420                 425                 430

Thr Ser Trp Arg Ser Asn Asp Glu Phe Ala Arg Glu Phe Leu Ala Gly
        435                 440                 445

Leu Asn Pro Val Met Ile Thr Arg Val Lys Phe Pro Ile Arg Ser Ser
    450                 455                 460

Leu Asp Pro Ala Glu Phe Gly Asp Pro Thr Ser Ala Ile Thr Lys Asp
465                 470                 475                 480

His Ile Glu Gly Ser Leu Glu Gly Leu Ser Val Glu Glu Ala Val Thr
                485                 490                 495

Ser Asn Arg Leu Phe Val Val Asp Tyr His Asp Ala Phe Leu Pro Phe
            500                 505                 510

Val Ala Lys Ile Asn Ala Gln Gln Asn Ser Ala Thr Tyr Ala Thr Arg
        515                 520                 525

Thr Leu Leu Phe Leu Ser Lys Asp Gly Ile Leu Lys Leu Leu Ala Ile
    530                 535                 540

Glu Leu Ala Leu Pro Pro Lys Thr Val Gly Glu Glu Arg Ile Thr Arg
545                 550                 555                 560

Val Leu Thr Thr Arg Lys Asp Asp Gln Leu Trp Lys Val Asn Trp Glu
                565                 570                 575

Trp Glu Leu Ala Lys Ala His Val Ser Asn Asn Asp Ile Thr Ala His
            580                 585                 590

Gln Val Phe Ser His Phe Ser Arg Cys His Ala Val Thr Glu Ala Val
        595                 600                 605

Ile Ile Cys Ser Asn Arg Asn Leu Ser Lys Leu His Pro Leu Met Gln
    610                 615                 620

Leu Leu Ala Pro His Phe Lys Ser Thr Leu Glu Ile Asn Arg Gln Ala

625                 630                 635                 640

Arg Ala Thr Leu Ile Ala Ala Gly Gly Ser Ile Glu Thr His Phe Thr
                645                 650                 655

Thr Arg Ala Tyr Ser Leu Glu Met Ala Ala Val Asn Tyr Lys Asp Thr
            660                 665                 670

Trp Thr Phe Glu Ser Gln Ala Leu Pro Thr Asp Leu Val Ala Arg Gly
        675                 680                 685

Met Ala Val Pro Asp Pro Asp Ser Pro His Gly Val Arg Leu Val Val
    690                 695                 700

Glu Asp Tyr Pro Tyr Ala Ala Asp Gly Leu Glu Leu Trp Gly Ala Leu
705                 710                 715                 720

Lys Ala Trp His Lys Glu Tyr Val Asp Ile Tyr Tyr Lys Asp Asp Ala
                725                 730                 735

Ala Val Leu Gln Asp Ser Glu Leu Met Thr Trp Trp Thr Glu Met Arg
            740                 745                 750

Glu Lys Ala His Glu Asp Lys Lys Asp Ser His Gly Trp Pro Glu Leu
        755                 760                 765

Asn Ser Lys Glu Ala Leu Val Asp Ile Leu Thr Thr Val Ile Trp Ile
    770                 775                 780

Pro Ser Cys Leu His Ala Ala Val Asn Phe Gly Gln Tyr Asp Phe Ala
785                 790                 795                 800

Gly Phe Met Pro His His Pro Thr Leu Thr Arg Arg Leu Leu Pro Glu
                805                 810                 815

His Gly Asn Glu Lys Asp Lys Ala Asp Phe Asn Lys Asn Pro Glu Lys
            820                 825                 830

Tyr Tyr Leu Thr Ser Ile Ser Asn Ile Asp Ser Thr Thr Thr Ala Met
        835                 840                 845

Ser Val Tyr Glu Val Leu Ser Ala His Cys Pro Ile Glu Glu Tyr Ile
    850                 855                 860

Gly Glu Arg Arg Gly Asn Trp Thr Asn Asn Glu Lys Val Leu Ala Ala
865                 870                 875                 880

Phe Lys Gly Phe Lys Glu Ser Val Asn Glu Ala Asp Ala Val Met Arg
                885                 890                 895

Ala Arg Asn Ala Asp Pro Lys Leu Arg Asn Arg Gly Gly Pro Val Lys

900                 905                 910

Met Pro Tyr Gln Leu Leu Arg Pro His Ser Lys Pro Gly Val Thr Ser
        915                 920                 925

Met Gly Val Pro Asn Ser Ile Thr Ile
    930                 935

<210>  16
<211>  715
<212>  PRT
<213>  Plexaura homomalla

<300>
<308>  TrEMBL/AAC47283
<309>  1996-11-01
<313>  (1)..(715)

<400>  16

Met Ala Lys Leu Leu Ile Ile Leu Ser Leu Phe Leu Thr Trp Gln Ile
1               5                   10                  15

Cys Ala Gly Phe Leu Leu Asn Asp Asn Leu Cys Lys Tyr Lys Lys Glu
            20                  25                  30

Ser Asn Val Phe Ser Lys Arg Ser Arg Leu Cys Asp Glu Asn Gln Arg
        35                  40                  45

Asp Cys Arg Arg Phe Leu Gly Trp Leu Ala Pro Lys Glu Glu Pro Gly
    50                  55                  60

Asp Asp Glu Tyr Tyr Asp Cys Cys Pro Gly Phe Thr Lys Lys Gly Arg
65                  70                  75                  80

Phe Gly Cys Gln Ile Lys Thr Cys Ile Val Leu Pro Gln Asp Val Asn
                85                  90                  95

Lys Phe Pro Leu Met Cys Pro Phe Glu Arg His Ala Asp Leu Val Leu
            100                 105                 110

Glu Asn Leu Phe Tyr Lys Leu Thr Thr Pro Pro Glu Asn Pro Leu Glu
        115                 120                 125

Tyr Pro Ala Glu Thr Ser Asn Ile Leu Glu Ala Val Pro Phe Pro Leu
    130                 135                 140

Leu Asn Gln Pro Ser Glu Lys Val Leu Leu Arg Ser Ala Leu Lys Pro
145                 150                 155                 160

Arg Leu Lys Glu Thr Glu Trp Leu Ile Arg Tyr Ile Gly Met Ser Thr
                165                 170                 175

Arg Asn Gly Ala Leu Ala Lys Lys Thr Ile Glu Asn Pro Asp Ile Lys

            180                 185                 190

Pro Phe Thr Asp Tyr Ala Asp Tyr Ala Gln Leu Leu Lys Ser Phe Arg
        195                 200                 205

Asp Glu Gln Ser Lys Asp Leu Ile Ser Leu Gln Ile Pro Gly Val Asn
    210                 215                 220

His Thr Phe Asp Leu Val Ser Met Glu Glu Tyr Met Arg Tyr Pro Ser
225                 230                 235                 240

Gln Glu Thr Pro Phe Lys Asn Ile Ile His Asn Asn Ile Trp Gln Asn
                245                 250                 255

Asp Asp Val Phe Thr Gln Gln Arg Leu Ala Gly Leu Asn Pro Met Ser
            260                 265                 270

Leu Arg Lys Met Thr Asn Glu Asp Phe Asn Arg Asp Phe Ile Asn Lys
        275                 280                 285

Thr Tyr Asp Trp Asp Ala Ala Met Gln Arg Ala Ser Gly Glu Asp Ser
    290                 295                 300

Thr Phe Ser Glu Met Ile Asp Ala Gly Arg Val Tyr Ile Val Glu Phe
305                 310                 315                 320

Ser Tyr Leu Tyr Asp Leu Ser Asn Val Glu Asp Leu Thr Glu Arg Asn
                325                 330                 335

Thr Thr Asp Arg Arg Glu Met Arg Gly Ser Arg Ala Pro Ile Cys Thr
            340                 345                 350

Phe Val Ser Ala Asn Val Gly Gly Val Arg Glu Leu Lys Pro Val Ala
        355                 360                 365

Ile Gln Thr Asp Gln Ser Ser Asp Ala Pro Val Phe Ser Pro Val Asp
    370                 375                 380

Gly Ser Lys Trp Leu Met Ala Lys Gln Glu Phe Gln Arg Ala Asp Ile
385                 390                 395                 400

Thr Tyr Ala Glu Ile Ile Glu His Leu Leu Lys Thr His Leu Leu Met
                405                 410                 415

Glu Pro Ile Cys Val Ile Met Arg Arg Thr Leu Ser Lys Phe His Pro
            420                 425                 430

Leu His Glu Ile Leu Lys Trp His Cys Arg Gly Leu Phe Ala Val Asn
        435                 440                 445

Ser Val Gly Ile Lys Ala Leu Leu Asn Pro Glu Glu Phe Leu His Lys

450                 455                 460

Leu Phe Ala Ile Gly His Ala Gly Gly Val Glu Leu Leu Asn Lys Ala
465                 470                 475                 480

Tyr Pro Asp Met Ser Trp Gly Val Thr Glu Phe Asp Lys Asn Leu Glu
                485                 490                 495

Arg Arg Gly Val Asp Ser Thr Asp Glu Ile Pro Tyr Phe Pro Tyr Arg
            500                 505                 510

Asp Asp Gly Leu Leu Ile Trp Lys Glu Val Ser Asn Phe Ser Arg Asp
        515                 520                 525

Tyr Ile Asn Leu Tyr Tyr Ser Asp Asp Ser Arg Val Ala Gly Asp Ser
    530                 535                 540

Glu Leu Gly Arg Phe Phe Asn Gln Leu Ser Ala Asp Gly Thr Gly Thr
545                 550                 555                 560

Asp Gly Gly Ile Gly Met Phe Lys Gly Phe Pro Ala Lys Ile Glu Thr
                565                 570                 575

Ile Gln Asp Leu Ile Asp Val Val Lys Arg Ile Val Phe Ile Pro Val
            580                 585                 590

Gln His His Ala Ile Asn Tyr Pro Val Ala Tyr Tyr Gly Gly Phe Val
        595                 600                 605

Pro Asn Leu Pro Thr Lys Leu Tyr Asp Asp Pro Arg Val Pro Pro Lys
    610                 615                 620

Glu Phe Gly Phe Asp Arg Leu Pro Gln Tyr His Val Ala Ala Glu Gln
625                 630                 635                 640

Val Ala Leu Ser Met Ser Leu Gly Ser Ile Arg Tyr Asp Val Leu Phe
                645                 650                 655

Asp Tyr Gly Lys His Leu Lys Asp Asp Arg Ala Lys Ala Ile Val Asn
            660                 665                 670

Lys Tyr His Glu Arg Leu Thr Gly Ser Ile Asn Asp Gln Ile Ala Ala
        675                 680                 685

Arg Asn Gln Glu Arg Leu Gln Asn Gly Lys Leu Ser Tyr Pro Tyr Leu
    690                 695                 700

Leu Pro Lys Trp Met Pro Asn Ser Ile His Thr
705                 710                 715

SEQUENCE LISTING

<110>  Universität Göttingen

<120>  Method of producing fatty acid hydroperoxides

<130>  U 7082

<160>  12

<170>  PatentIn version 3.3

<210>  1
<211>  630
<212>  PRT
<213>  Nostoc sp. PCC73102

<400>  1

Met Thr Ala Leu Ser Pro Asp His Ser Ile Ser Ser Ser Ser Thr His
1               5                   10                  15

Thr Leu Asp Ile Ala Arg Gln Glu Tyr Gln Tyr Asn Tyr Asn His Ile
            20                  25                  30

Pro Ser Ile Ala Met Val Asp Arg Leu Ser Ile Thr Glu Glu Phe Thr
        35                  40                  45

Thr Asn Trp Tyr Phe Leu Leu Ala Gln Gln Leu Arg Val Leu Phe Ile
    50                  55                  60

Asn Thr Leu Ile Val Asn Arg Gly Asn Gln Asp Ser Lys Ser Ile Arg
65                  70                  75                  80

Asp Asp Val Glu Arg Phe Ile Leu Glu Ala Leu Leu Lys Gly Ala Val
                85                  90                  95

Pro Ala Arg Ile Ser Ile Leu Ala Arg Ile Leu Gln Ile Ile Pro Gln
            100                 105                 110

Leu Leu Leu Lys Glu Ile Ser Lys Asp Phe Arg Glu Leu Asp Asp Leu
        115                 120                 125

Phe His Ser Ile Leu Lys Glu Asn Gly Leu Ala Ile Leu Arg Asp Ala
    130                 135                 140

Leu Lys Arg Ile Ile Thr Leu Leu Tyr Glu Gly Gln Pro Thr Gly His
145                 150                 155                 160

Ala Thr Ser Leu Lys Asp Tyr Glu Asn Leu Phe Pro Val Ile Ser Leu
                165                 170                 175

Pro Ala Ile Ala Lys Thr Tyr Gln Glu Asp Glu Val Phe Ala Tyr Met
            180                 185                 190

Arg Val Ala Gly Tyr Asn Pro Val Thr Ile Lys Arg Val Thr Thr Leu
        195                 200                 205

Ser Asp Arg Phe Pro Val Thr Asp Glu His Tyr Gln Ala Val Met Gly
    210                 215                 220

Thr Asp Asp Ser Leu Ala Ala Ala Gly Ile Glu Gly Arg Leu Tyr Leu
225                 230                 235                 240

Ala Asp Tyr Lys Ile Leu Asp Gly Ala Ile Asn Gly Thr Phe Pro His
                245                 250                 255

Glu Gln Lys Tyr Leu Tyr Ala Pro Leu Ala Leu Phe Ala Leu Pro Lys
            260                 265                 270

Gly Ser Asp Pro Thr Arg Leu Leu Arg Pro Val Ala Ile Gln Cys Gly
        275                 280                 285

Gln Thr Pro Gly Pro Asp Tyr Pro Ile Val Thr Pro Asn Ser Gly Lys
    290                 295                 300

Tyr Ala Trp Leu Phe Ala Lys Thr Ile Val Gln Ile Ala Asp Ala Asn
305                 310                 315                 320

Ile His Glu Ala Val Thr His Leu Ala Arg Thr His Leu Leu Val Gly
                325                 330                 335

Val Phe Ala Ile Ala Thr Ala Arg Gln Leu Pro Leu Thr His Pro Leu
            340                 345                 350

Arg Ile Leu Leu Arg Pro His Phe Asp Ser Thr Leu Ala Ile Asn Asp
        355                 360                 365

Ala Ala Gln Arg Ile Leu Ile Ala Pro Gly Gly Gly Val Asp Arg Leu
    370                 375                 380

Leu Ser Ser Ser Ile Asp Asn Ser Arg Val Leu Ala Val Leu Gly Leu
385                 390                 395                 400

Gln Ser Tyr Ser Phe Asn Ser Thr Ile Leu Pro Asn Gln Phe Gln Gln
                405                 410                 415

Arg Gly Val Asp Asp Pro Asn Leu Leu Pro Ile Tyr Pro Tyr Arg Asp
            420                 425                 430

Asp Ala Leu Leu Ile Trp Asn Ala Ile His Gln Trp Val Trp Asp Tyr
        435                 440                 445

Leu Asn Leu Tyr Tyr Thr Thr Asp Glu Asp Ile Gln Lys Asp Arg Ala
    450                 455                 460

Leu Gln Ala Trp Ala Ala Glu Ile Pro Ala Tyr Asp Gly Gly Arg Ile
465                 470                 475                 480

Pro Asp Phe Gly Glu Asp Gly Gly Ile Lys Thr Leu Asn Tyr Leu Ile
                485                 490                 495

Asp Ala Thr Thr Leu Ile Ile Phe Thr Ala Ser Ala Gln His Ala Ala
            500                 505                 510

Val Asn Phe Pro Gln Lys Asp Leu Met Gly Tyr Ala Ala Ala Ile Pro
        515                 520                 525

Leu Ala Gly Tyr Leu Pro Ala Ser Thr Leu Lys Arg Glu Val Thr Glu
    530                 535                 540

Gln Asp Tyr Leu Asn Leu Leu Pro Pro Leu Asp Gln Ala Gln Arg Gln
545                 550                 555                 560

Tyr Asn Leu Leu Ser Leu Leu Gly Ser Val Tyr Tyr Asn Lys Leu Gly
                565                 570                 575

Glu Tyr Glu Gln Gly Tyr Phe Thr Asp Glu Lys Val Lys Pro Leu Leu
            580                 585                 590

Gln Ala Phe Gln Ser His Leu Gln Gln Val Glu Asn Thr Ile Lys Gln
        595                 600                 605

Arg Asn Leu His Arg Pro Pro Tyr Glu Tyr Leu Leu Pro Ser Lys Ile
    610                 615                 620

Pro Gln Ser Ile Asn Ile
625                 630

<210>  2
<211>  547
<212>  PRT
<213>  Nostoc sp. PCC73102

<400>  2

Met Lys Pro Tyr Leu Pro Gln Asn Asp Pro Asp Pro Thr Lys Arg Gln
1               5                   10                  15

Ile Leu Leu Glu Arg Asn Gln Gly Glu Tyr Glu Phe Asp Tyr Asp Phe
            20                  25                  30

Leu Val Pro Met Ala Met Leu Lys Asn Val Pro Ser Ile Glu Asn Phe
        35                  40                  45

Ser Thr Lys Tyr Ile Ala Glu Arg Thr Leu Glu Thr Ala Glu Leu Pro
    50                  55                  60

Ile Asn Met Leu Ala Val Lys Thr Arg Ser Leu Trp Asp Pro Leu Asp
65                  70                  75                  80

Glu Leu Gln Asp Tyr Glu Asp Tyr Phe Pro Val Leu Pro Lys Pro Asn
                85                  90                  95

Ile Ile Lys Thr Tyr Gln Ser Asp Asp Ser Phe Cys Glu Gln Arg Leu
            100                 105                 110

Cys Gly Ala Asn Pro Phe Val Leu Arg Arg Ile Glu Gln Met Pro Asp
        115                 120                 125

Gly Phe Ala Phe Thr Ile Leu Glu Leu Gln Glu Lys Phe Gly Asp Ser
    130                 135                 140

Ile Asn Leu Val Glu Lys Leu Ala Asn Gly Asn Leu Tyr Val Ala Asp
145                 150                 155                 160

Tyr Arg Ala Leu Ala Phe Val Lys Gly Gly Ser Tyr Glu Arg Gly Lys
                165                 170                 175

Lys Phe Leu Pro Thr Pro Ile Ala Phe Phe Cys Trp Arg Ser Ser Gly
            180                 185                 190

Phe Ser Asp Arg Gly Gln Leu Val Pro Ile Val Ile Gln Ile Asn Pro
        195                 200                 205

Ala Asp Gly Lys Gln Ser Gln Leu Ile Thr Pro Phe Asp Asp Pro Leu
    210                 215                 220

Thr Trp Phe His Ala Lys Leu Cys Val Gln Ile Ala Asp Ala Asn His
225                 230                 235                 240

His Glu Met Ser Ser His Leu Cys Arg Thr His Phe Val Met Glu Pro
                245                 250                 255

Phe Ala Ile Val Thr Ala Arg Gln Leu Ala Glu Asn His Pro Leu Ser
            260                 265                 270

Leu Leu Leu Lys Pro His Phe Arg Phe Met Leu Ala Asn Asn Asp Leu
        275                 280                 285

Ala Arg Lys Arg Leu Ile Ser Arg Gly Gly Pro Val Asp Glu Leu Leu
    290                 295                 300

Ala Gly Thr Leu Gln Glu Ser Leu Gln Ile Val Val Asn Ala Tyr Thr
305                 310                 315                 320

Glu Trp Ser Leu Asp Gln Phe Ser Leu Pro Thr Glu Leu Lys Asn Arg
                325                 330                 335

Gly Met Asp Asp Pro Asp Asn Leu Pro His Tyr Pro Tyr Arg Asp Asp
            340                 345                 350

Gly Leu Leu Leu Trp Asn Ala Ile Lys Lys Phe Val Ser Glu Tyr Leu
        355                 360                 365

Gln Ile Tyr Tyr Lys Thr Pro Gln Asp Leu Ala Glu Asp Leu Glu Leu
    370                 375                 380

Gln Ser Trp Val Gln Glu Leu Val Ser Gln Ser Gly Gly Arg Val Lys
385                 390                 395                 400

Gly Ile Ser Asp Arg Ile Asn Thr Leu Asp Gln Leu Val Asp Ile Ala
                405                 410                 415

Thr Ala Val Ile Phe Thr Cys Gly Pro Gln His Ala Ala Val Asn Tyr
            420                 425                 430

Ser Gln Tyr Glu Tyr Met Thr Phe Met Pro Asn Met Pro Leu Ala Ala
        435                 440                 445

Tyr Lys Gln Met Thr Ser Glu Gly Thr Ile Pro Asp Arg Lys Ser Leu
    450                 455                 460

Leu Ser Phe Leu Pro Pro Ser Lys Gln Thr Ala Asp Gln Leu Ser Ile
465                 470                 475                 480

Leu Phe Ile Leu Ser Ala Tyr Arg Tyr Asp Arg Leu Gly Tyr Tyr Asp
                485                 490                 495

Asp Lys Phe Leu Asp Pro Glu Ala Gln Asp Val Leu Ala Lys Phe Gln
            500                 505                 510

Gln Glu Leu Asn Glu Ala Glu Arg Glu Ile Glu Leu Asn Asn Lys Ser
        515                 520                 525

Arg Leu Ile Asn Tyr Asn Tyr Leu Lys Pro Arg Leu Val Thr Asn Ser
    530                 535                 540

Ile Ser Val
545

<210>  3
<211>  1893
<212>  DNA
<213>  Nostoc sp. PCC73102

<400>  3
atgactgctt tatcaccaga tcattcaatc agttcatcaa gtactcatac tttagatata      60

gctaggcaag agtatcaata taactacaac catattccat ctattgcgat ggtggatcgg     120

ctttctatta cggaagagtt caccactaac tggtattttt tattagccca gcagttacga     180

gttcttttta tcaatacctt gattgtcaac agaggcaatc aagattctaa atcgattcgt     240

gatgatgtcg aaagatttat tttagaagcc ttgctcaagg gagcagtacc agcccgaatc     300

agcattctgg caagaatcct gcaaattatc cctcagcttt tgctcaaaga gatatctaag 360

gattttagag aactcgacga tctttttcat tctattctga aagaaaacgg acttgcgatc 420

ctcagagatg ctctaaagag gataattacc cttctatatg aaggacagcc tacaggacat 480

gcaaccagtc tcaaggacta cgaaaatttg tttccggtga ttagtctacc agccatcgct 540

aaaacttatc aggaagatga agtatttgcc tacatgagag tagctggcta caatcccgtc 600

acgatcaagc gagtaactac tctaagcgat cgcttcccag tcacagacga acattaccaa 660

gcggtgatgg gaactgacga ttcattagca gcagcaggga ttgaaggcag gctctactta 720

gctgactata aaattttaga tggtgcaatc aacggtacat tcccacacga gcaaaaatat 780

ctctatgctc ccctagcact atttgcctta cccaaaggtt cagatcccac ccgtctattg 840

cgtccggtag ccattcaatg cggtcagacc ccaggcccag attatccaat tgttactcct 900

aactccggta aatacgcttg gctttttgcc aaaacgattg tccaaatagc agatgctaac 960

atccacgaag ctgttactca cctagcaaga actcatcttt tagttggtgt ttttgcgatc 1020

gcaactgctc gacaattgcc actcacacat cccctcagaa tcctgctgcg cccgcatttt 1080

gatagcacct tagcaattaa cgatgccgcc caacggattc tcattgctcc aggcggtggt 1140

gtcgatagat tactctcatc atcaatcgat aactctcgcg ttttagcagt gctagggttg 1200

caaagctata gttttaacag caccatctta cctaatcaat tccaacagcg cggtgtagac 1260

gatcctaatc tattgcctat ttatccttac cgggatgatg ccctactaat ctggaatgcc 1320

attcatcaat gggtttggga ctacctgaac ctttactaca ccacggatga agacattcaa 1380

aaagacagag cattgcaagc gtgggcagcc gaaatcccag cttacgatgg tggtcgtatc 1440

cccgattttg gcgaagatgg gggcatcaaa acgctaaatt acctgattga tgccactaca 1500

ttaattattt ttactgccag tgcccaacat gcagccgtta actttccgca aaaagatttg 1560

atgggctacg ccgcagccat accattggca ggttatttac cagcctcaac tctcaaaaga 1620

gaagttaccg agcaagacta ccttaatctg ctcccccctt tagatcaggc acaacggcaa 1680

tacaacctac tcagcttatt agggtctgta tactacaaca agctgggcga gtatgagcaa 1740

ggatacttca cagacgagaa agtaaaacca ttgttacagg cattccaaag tcatcttcag 1800

caggtagaaa ataccatcaa gcagcgtaat ttgcaccgcc caccctatga gtatctcctt 1860

ccttctaaaa ttcctcagag catcaatatc tga 1893

<210> 4
<211> 1644

<212>  DNA
<213>  Nostoc sp. PCC73102

<400>  4
atgaaaccat acctccctca gaatgatcct gaccctacaa aacgtcaaat attgctagag      60

agaaatcaag gggagtatga atttgattac gactttttgg tacctatggc aatgctaaaa     120

aatgtacctt ctatagaaaa cttttcaact aagtatattg ctgaacggac attagagaca     180

gcagaactgc ctataaatat gttagccgtt aaaacccgtt ctttatggga ccctttagat     240

gaattgcaag actatgaaga ctattttcca gttttgccta aacctaatat tatcaaaaca     300

taccaaagtg atgactcttt ttgtgagcaa cggctttgtg gggcaaatcc ttttgtttta     360

cgtcgaattg agcagatgcc agatggcttc gcctttacca ttttagaatt gcaagaaaaa     420

tttggtgact ctattaactt agtagaaaaa cttgcgaatg gaaatttata tgtagctgat     480

tacagagcgc ttgcgtttgt taaaggaggt agttatgaaa gaggtaagaa gtttttacca     540

acccctatag ctttcttttg ttggcgcagt tctggtttta gcgatcgcgg tcaactagta     600

ccgattgtta tccaaatcaa ccccgcagat ggcaaacaga gccagctaat tacacctttc     660

gatgaccctt taacctggtt tcatgccaag ctttgtgttc aaattgctga tgctaaccat     720

catgaaatga gtagccatct gtgtcgaact cactttgtta tggaaccctt tgctattgtc     780

acagcccgtc aactagccga gaaccatccc cttagcttac tgctaaaacc ccacttccgt     840

ttcatgttgg ctaataatga cttggctcgt aagcgcctaa ttagtagagg tgggcctgtt     900

gacgaattgc tagccggaac tctgcaagag tcattgcaaa ttgtcgttaa cgcatataca     960

gaatggagct tagatcagtt ttccttacct actgaactaa aaaatcgggg tatggatgat    1020

ccagacaact tacctcacta tccctatcga gacgatggct tattattgtg gaatgccatt    1080

aaaaagtttg tgtctgaata cttgcagata tactacaaaa ctccccaaga tttagcagaa    1140

gacttggaat tacaaagttg ggtgcaggaa ttagtttccc aatcaggcgg acgagtcaag    1200

ggtattagcg accgcatcaa cacattagac caattagttg atattgctac tgcggttatc    1260

ttcacctgtg ggccgcaaca cgctgctgtt aactactcac aatatgaata tatgactttc    1320

atgccaaata tgcctcttgc tgcttataaa caaatgacat cagaaggcac tattcctgac    1380

cgtaaaagtc tattatcatt tctgccaccg tcaaagcaaa ctgctgacca attatcgatt    1440

ttatttatcc tgtcggccta ccgttatgac agattagggt actacgatga taaattttta    1500

gacccagagg ctcaggatgt tttagctaaa ttccagcagg agttgaatga agcagagcgg    1560

gaaattgagt tgaataacaa gagtcgttta ataaattaca actatctcaa accaaggctt    1620

gtgactaata gtattagcgt gtaa                                              1644

<210>  5
<211>  36
<212>  DNA
<213>  Artificial

<220>
<223>  Primer NpLOX40F

<400>  5
atgactgctt tatcaccaga tcattcaatc agttca                                  36

<210>  6
<211>  28
<212>  DNA
<213>  Artificial

<220>
<223>  Primer NpLOX40R

<400>  6
tcagatattg atgctctgag gaatttta                                           28

<210>  7
<211>  27
<212>  DNA
<213>  Artificial

<220>
<223>  Primer NpLOX50F

<400>  7
atgaaaccat acctccctca gaatgat                                            27

<210>  8
<211>  27
<212>  DNA
<213>  Artificial

<220>
<223>  Primer NpLOX50R

<400>  8
tcacacgcta atactatttg acacaag                                            27

<210>  9
<211>  34
<212>  DNA
<213>  Artificial

<220>
<223>  NpLOX1F

<400>  9
cgcggatcca tgactgcttt atcaccagat catt                                    34


<210>  10
<211>  34
<212>  DNA
<213>  Artificial

<220>
<223>  NpLOX1R

<400>  10
tgcactgcag tcagatattg atgctctgag gaat                                    34


<210>  11
<211>  32
<212>  DNA
<213>  Artificial

<220>
<223>  NpLOX2a

<400>  11
cggatccatg aaaccatacc tccctcagaa tg                                      32


<210>  12
<211>  34
<212>  DNA
<213>  Artificial

<220>
<223>  NpLOX2b

<400>  12
gctgcagtta cacgctaata ctattagtca caag                                    34

## Description

The present invention relates to methods for producing fatty acid hydroperoxides. In particular, this invention relates to the production of C18 hydroperoxy polyunsaturated fatty acids, catalyzed by a specific 13-lipoxygenase (LOX) originating from a cyanobacterium. Further, the invention relates to methods of producing flavours, odours and aromas using the 13-LOX derived hydroperoxy fatty acids as starting compounds. The inventive 13-LOX is useful for the production of hydroperoxy fatty acids at a large-scale, and their further processing into flavour and fragrance substances.

The formation of oxidized polyunsaturated fatty acids (PUFAs) is one of the main reactions in lipid alteration. The initial formation of hydroperoxides may occur either by chemical oxidation or by the action of enzymes such as lipoxygenases (LOXs) or α-dioxygenases. The metabolism of PUFAs via the LOX-catalyzed step and the subsequent reactions are collectively named LOX pathway. LOXs (linoleate:oxygen oxidoreductases, EC 1.13.11.12) constitute a large gene family of non-heme iron-containing fatty acid dioxygenases, which are ubiquitous in plants and animals. LOXs catalyze the regio- and stereospecific dioxygenation of PUFAs containing at least one (1Z,4Z)-pentadiene system. Thus, substrates for LOXs are for example linoleic acid (LA, 18:2), α-linolenic acid (LeA or ALA, 18:3), or arachidonic acid (AA, 20:4).

LOXs can also peroxidize fatty acids esterified in glycerolipids such as phospholipids and apparently glycolipids, but free fatty acids are the preferred substrate for most LOXs at least in vitro.

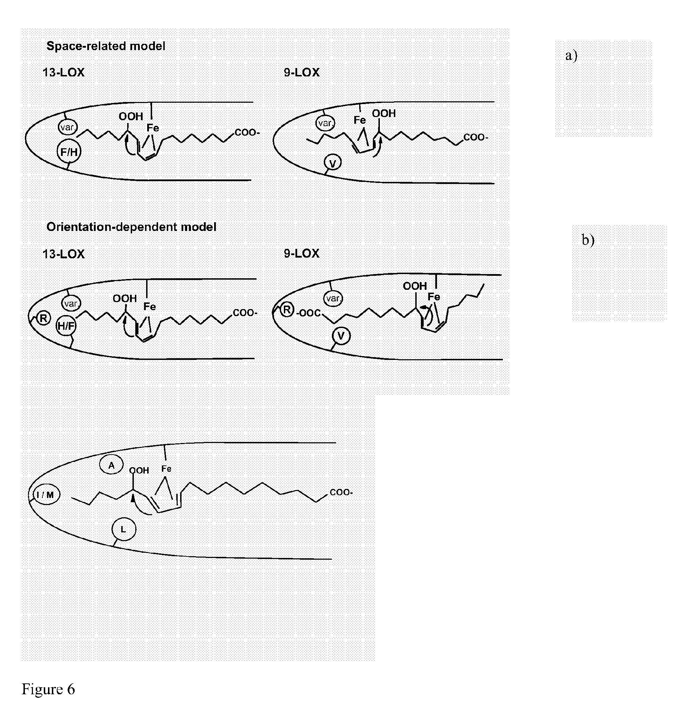

In mammals, LOXs are classified according to their positional specificity of AA oxygenation. Because AA is either not present in flowering plants or is a minor constituent of their cellular lipids, plant LOXs are classified into 9- and 13-LOXs with respect to their positional specificity of LA oxygenation. LA is oxygenated either at carbon atom 9 (9-LOX) or at carbon atom 13 (13-LOX) of the hydrocarbon backbone of the fatty acid leading to two groups of compounds, the (9S)-hydroperoxy- and the (13S)-hydroperoxy derivatives of LA. LOXs are versatile catalysts because they are multifunctional enzymes, catalyzing at least three different types of reactions: (i) dioxygenation of lipid substrates (dioxygenase reaction), (ii) secondary conversion of hydroperoxy lipids (hydroperoxidase reaction), and (iii) formation of epoxy leukotrienes (leukotriene synthase reaction). Under physiological conditions the dioxygenation reaction is most prevalent in plants and their major substrates are C18-PUFAs. The two different regioisomers of hydroxyperoxy PUFAs may be determined by two independent properties of catalysis. (i) Selectivity in the initial hydrogen removal: In LeA this is possible either at C-11 and/or C-14, but comparison of various plant LOXs as well as site-directed mutagenesis studies revealed that only one double-allylic methylene group at C-11 in LA or LeA is used. (ii) Selectivity in the site of oxygen insertion via rearrangement of the intermediate fatty acid radical. Consequently, when hydrogen is abstracted at C-11, molecular oxygen can be introduced at position [+2] or [-2] leading to dioxygen insertion at C-13 or C-9. Again, in principle, two models are used to explain the underlying reaction mechanism of positional specificity of LOXs (Figure 6). Based on data on mammalian LOXs, a space-related hypothesis was established (Figure 6a). Here, the fatty acid substrate penetrates the active site generally with its methyl end first. Then the depth of the substrate-binding pocket determines the site of hydrogen abstraction, and the positional specificity of molecular oxygen insertion depends on this position. However, in plant LOX reactions only one double allylic methylene group in the natural substrates LA and LeA seems to be accessible, rendering the space-related hypothesis unlikely. According to a second hypothesis (Figure 6b), the substrate orientation is regarded as the key step in the determination of the position of dioxygen insertion, which then leads to varying regiospecificities of different isozymes: In the case of 13-LOXs, the active site is penetrated again by the substrate using the methyl end first, whereas with 9-LOXs the substrate is forced into an inverse orientation favouring a penetration with its carboxy group first. Consequently, a radical rearrangement at either [+2] or [-2], respectively, may be facilitated in both cases by the same mechanism within the active site.

Oxygenation of naturally occurring PUFAs may proceed enzymatically and/or chemically. Both reactions lead to the formation of hydroperoxy PUFAs but there are important differences between the two processes. The most important difference is the specificity of the product pattern. Non-enzymatic lipid peroxidation leads to an unspecific product mixture consisting of various positional and optical isomers. In contrast, during LOX reaction PUFAs are usually oxygenated to one specific product isomer which exhibits a high degree of optical purity. Thus, for large-scale preparation of structurally well-defined oxygenated PUFAs LOXs exhibiting different positional specificities may be used.

Both hydroperoxide derivatives serve as substrates for numerous metabolic reactions: They may be cleaved to aldehydes and ω-oxo acids, and the 13-hydroperoxide of LeA may serve as a precursor for the synthesis of jasmonic acid. The metabolism of PUFAs via this LOX-catalyzed step and the subsequent reactions are collectively named LOX pathway and the metabolites that derive therefrom are called oxylipins.

By cleavage of the hydroperoxides to the corresponding aldehydes, green notes, which include n-hexanal, hexan-1-ol, 2-(E)-hexen-1-al, 2-(E)-hexen-1-ol and 3-(Z)-hexen-1-ol, may be produced which are widely used in flavors, particularly fruit flavors, to impart a fresh green character. Furthermore, green notes are essential for fruit aroma and are used extensively in the aroma industry. Currently, homogenized soybeans provide the cheapest source of 13-LOX (see e.g. US 5,464,761; US 6,780,621). However, the reaction broth of these reactions contains a lot of solids introduced by the milled soybeans. The solids and the increased viscosity are often detrimental during the subsequent processing. Therefore, the use of a recombinant microorganism such as *E. coli* expressing a 13-LOX may provide an alternative to the soybean derived 13-LOX.

Thus, there is a strong interest in identifying and characterizing useful LOXs which show high regiospecificity and which can be used to produce well-defined oxygenated PUFAs. In particular, there is a need for LOXs having specificity for C-13 which can be easily produced by recombinant DNA technology in order to be used in large-scale processes for the production of 13-LOX-derived PUFA hydroperoxides. In this respect, a prokaryotic 13-LOX enzyme seems to be particularly useful, since its expression in bacterial cells like e.g. *Escherichia coli* should be much easier than the expression of a plant or mammalian LOX.

However, so far no prokaryotic LOX with C-13-specificity seems to be known in the prior art. It is thus an object of the present invention to identify a prokaryotic LOX enzyme with C-13-specificity and to develop a process using such an enzyme in the production of 13-LOX-derived oxygenated PUFAs.

LOX products can be metabolized further and are known as signalling substances that play a role in plant development as well as in plant responses to wounding and pathogen attack. Apart from accumulating data in mammals, flowering and non-flowering plants, information on the relevance of lipid peroxide metabolism in prokaryotic organisms is scarce.

The above-mentioned objects of the invention are solved by the method according to claim 1. Preferred embodiments are represented by the subject-matter of the sub-claims.

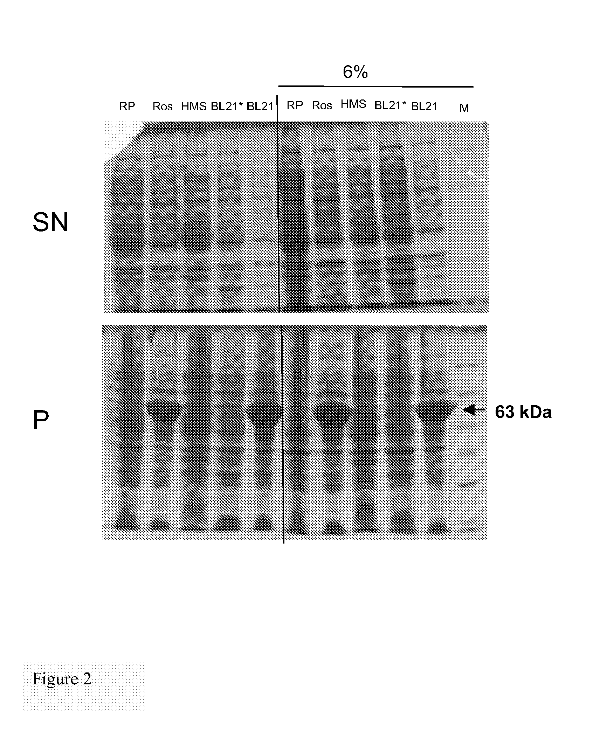

The inventors aimed to isolate and characterize LOXs and oxylipin patterns from cyanobacterial origin. DNA isolated from *Nostoc punctiforme* strain PCC73102 yielded sequences for at least two different LOXs. These have been cloned as cDNAs and named NpLOX1 and NpLOX2. Both proteins were identified as linoleate 13-LOXs by expression in *E. coli.* Analysis of the expression (Fig. 2) showed that both LOX proteins are expressed at a very high level in *E.coli* cells. This is in contrast to the finding that plant derived LOXs cannot be strongly overexpressed in bacteria and shows the advantages of a bacterial derived 13-LOX over plant derived 13-LOX. NpLOX1 showed a broad pH optimum ranging from pH 4.5 to pH 8.5 with a maximum at pH 8.0 and LeA was the preferred substrate. Bacterial extracts contain more 13-lipoxygenase-derived hydroperoxides in stressed than in non stressed cells with a 30-fold excess of non esterified over esterified oxylipins. 9-Lipoxygenase-derived derivatives were not detectable. 13-LOX-derived hydroperoxides in esterified lipids were present at almost equal amounts compared to non esterified hydroperoxides in non stressed cells. These results suggest that 13-LOXs acting on free fatty acids dominate in *Nostoc punctiforme* strain PCC73102 and that the present invention for the first time provides a bacterial regiospecific 13-LOX.

The present invention relates to a method of producing polyunsaturated fatty acid hydroperoxides from polyunsaturated fatty acids by use of a protein having the enzymatic activity of a prokaryotic 13-LOX.

The invention further relates to a method for producing a bacterial 13-LOX with specificity towards PUFAs, especially towards C18 PUFAs and most especially towards LeA and to its utilization for hydroperoxidation of PUFAs, especially of C18 PUFAs and most especially of LeA at carbon atom 13. The inventive LOX can be easily prepared in a suitable host organism at a large scale and makes it possible to produce specific 13-LOX derived C18 hydroperoxy PUFAs, especially hydroperoxy LeA, also at large scale.

C18 PUFAs and in particular LeA or their natural precursors are incubated with the inventive 13-LOX, e.g. in pure form or in form of a host organism extract or with intact microbial cells, under appropriate conditions. Hydroperoxidation of the C18 PUFAs and in particular of LeA is then effected specifically at position 13.

The 13-LOX protein used in the method according to the invention is encoded by a nucleic acid sequence selected from the group consisting of:
a) nucleic acid sequences comprising a nucleotide sequence encoding a protein with the amino acid sequence depicted in SEQ ID NOs: 1 or 2, or comprising a fragment of said nucleotide sequence, wherein said fragment is sufficient to code for a protein having the enzymatic activity of a 13-lipoxygenase,
b) nucleic acid sequences comprising the nucleotide sequence depicted in SEQ ID NOs: 3 or 4, or comprising a fragment of said nucleotide sequence, wherein said fragment is sufficient to code for a protein having the enzymatic activity of a 13-lipoxygenase,
c) nucleic acid sequences comprising a nucleotide sequence which hybridizes to a complementary strand of the nucleotide sequence from a) or b) under stringent conditions, or comprising a fragment of said nucleotide sequence, wherein the fragment is sufficient to code for a protein having the enzymatic activity of a 13-lipoxygenase, and
d) nucleic acid sequences comprising a nucleotide sequence which shows at least 60 % identity to the nucleotide sequence from a), b) or c), or comprising fragments of said nucleotide sequence, wherein the fragment is sufficient to code for a protein having the enzymatic activity of a 13-lipoxygenase.

The term "polyunsaturated fatty acids (PUFAs)" refers to fatty acids with at least 2 double bonds. "Fatty acid hydroperoxides" are formed by oxidation of unsaturated fatty acids.

The term "natural precursors of fatty acids" refers to oils from different organisms which contain a considerable amount of the fatty acid which is to be converted by the 13-LOX to the corresponding hydroperoxide. For example, linseed oil may be used as a natural precursor of LeA and sunflower oil may be used as a natural precursor of LA.

The 13-LOX enzymes according to the present invention may be used in various systems.

First, recombinant microbial cells (e.g. E.coli) may be lysed, e.g. by sonification, osmotic shock or by the use of cell lysis kits (e.g. CelLytic^{™} of Sigma) and then be directly added to a solution of fatty acids purged with oxygen to form the fatty acid hydroperoxides at the optimal pH.

Second, the hydroperoxides may be produced in vivo in a transgenic cell or a transgenic organism such as microbial cells, plants, plant cell cultures, cyanobacteria. Optionally, these transgenic organisms may also overexpress an enzyme acting on the fatty acid hydroperoxide such as hydroperoxide lyase. In case of such a coexpression, the corresponding aldehydes may be produced from the hydroperoxides immediately after the latter are formed.

Finally, the 13-LOX may be purified from the transgenic organism. Preferably, the nucleotide sequence coding for the 13-LOX is cloned into a vector which contains a nucleotide sequence coding for an affinity tag such as cellulose binding protein, maltose binding protein, glutathione-S-transferase or a His-tag. After transcription and translation of the DNA a fusion protein is formed which can then be easily purified by means of affinity chromatography. Vectors and methods which are useful for the production and purification of the fusion protein are well-known to those skilled in the art. After the purification, the affinity tag may be removed by the action of a protease such as Factor X. However, such affinity tags provide increased activity of the recombinant fusion protein due to improved protein folding/solubility etc. These and other tags also allow the immobilisation of the fusion protein on cheap matrices such as amylose, cellulose, which can be used in reactors for the stabilization and recycling of the recombinant enzyme. Two general examples for purifying the LOX enzymes by affinity chromatography are given below.

The method according to the invention for expressing the 13-LOX in a transgenic organism causes an increase of the 13-LOX content in a transgenic organism, e.g. a transgenic plant and/or plant cell or a transgenic bacterial cell or a transgenic yeast cell as compared to the corresponding wild type cell. This increase in content is at least 5%, preferably at least 20%, also preferably at least 50%, especially preferably at least 100%, also especially preferably at least by the factor of 5, particularly preferably at least by the factor of 10, also particularly preferably at least by the factor of 50, and most preferably at least by the factor of 100.

According to the invention, the term "wild type" is to be understood as the respective non genetically modified parent organism.

The term "fragments of DNA," as it is used herein, is to be understood as DNA segments encoding a protein having the activity of a LOX, wherein the proteins encoded by the DNA segments essentially have the same LOX activity as the proteins encoded by the complete DNA sequence, and the production of polyunsaturated fatty acid hydroperoxides can be achieved with these fragments.

The term "fragments of the protein," as it is used herein, indicates protein segments having the activity of the specific 13-LOX according to the invention, wherein the protein segments essentially have the same LOX activity as the full length protein, and the production of polyunsaturated fatty acid hydroperoxides can be achieved with these fragments.

Essentially the same enzymatic activity of the LOX used in the method according to the invention means that the enzymatic activity as compared to the enzymes encoded by the nucleic acid sequence with SEQ ID No. 3 or SEQ ID No. 4, and their derivatives, is still at least 50%, preferably at least 60%, especially preferably at least 70%, and particularly preferably at least 80%, and most preferably at least 90%. LOX enzymes with essentially the same enzymatic activity are thus also capable of producing polyunsaturated fatty acid hydroperoxides in transgenic bacterial cells or transgenic plants.

The LOX activity can be determined by incubating the purified enzyme or extracts from host cells or a host organism with a fatty acid substrate such as LeA under appropriate conditions and analysis of the reaction products, e.g. by oxygen electrode, spectrophotometer or HPLC analysis. Prior to the analysis the reaction products may be reduced to hydroxy fatty acids. Details about enzyme activity assays and analysis of the reaction products are given below in the Examples.

The term "nucleic acid (molecule)", as it is used herein, comprises furthermore in a preferred embodiment the untranslated sequence located at the 3' and at the 5' end of the encoding gene region: at least 500, preferably 200, especially preferably 100 nucleotides of the sequence upstream of the 5' end of the encoding region, and at least 100, preferably 50, especially preferably 20 nucleotides of the sequence downstream of the 3' end of the encoding gene region.

An "isolated" nucleic acid molecule is separated from other nucleic acid molecules present in the natural repository of nucleic acids. An "isolated" nucleic acid preferably has no sequences naturally flanking the nucleic acid in the genomic DNA of the organism from which the nucleic acid originates (e.g. sequences located at the 5' and 3' ends of the nucleic acid). In various embodiments the isolated LOX molecule can contain, for example, less than approximately 5 kb, 4 kb, 3 kb, 2 kb, 1 kb, 0,5 kb, or 0,1 kb of nucleotide sequences which naturally flank the nucleic acid molecule in the genomic DNA of the cell from which the nucleic acid originates. All nucleic acid molecules mentioned herein can be e.g. RNA, DNA, or cDNA.

The nucleic acid molecules used in the method, such as a nucleic acid molecule with a nucleotide sequence of SEQ ID No. 3 or SEQ ID No. 4 or a part thereof, can be isolated using standard molecular biological techniques and the sequence information provided herein. With the aid of comparison algorithms, which can be found e.g. on the NCBI homepage under http://www.ncbi.nlm.nih.gov, a homologous sequence, for example, or homologous, conserved sequence regions can also be identified on DNA or amino acid level. Essential parts of this sequence, or the entire homologous sequence can be used as a hybridization probe using standard hybridization techniques (as described in Sambrook et al., vide supra) for isolating additional nucleic acid sequences from other organisms which are useful in the method by means of screening of cDNA and/or genomic libraries. Moreover, a nucleic acid molecule comprising a complete sequence of SEQ ID No. 3 or SEQ ID No. 4 or a part thereof, can be isolated by means of a polymerase chain reaction, using oligonucleotide primers on the basis of the sequences given herein or parts thereof (e.g. a nucleic acid molecule comprising the complete sequence or a part thereof can be isolated by means of polymerase chain reaction using oligonucleotide primers which have been generated based on this same sequence). For example, mRNA can be isolated from cells (e.g. by means of the guanidinium thiocyanate extraction method of Chirgwin et al. (1979) Biochemistry 18: 5294 - 5299), and cDNA can be produced from it by means of reverse transcriptase (e.g. Moloney-MLV reverse transcriptase, available from Gibco/BRL, Bethesda, MD, or AMV reverse transcriptase, available from Seikagaku Amerika, Inc., St. Petersburg, FL). Synthetic oligonucleotide primers for amplification by means of polymerase chain reaction can be generated based on one of the sequences shown in SEQ ID No. 3 or SEQ ID No. 4, or with the aid of the amino acid sequences shown in SEQ ID No. 1 or SEQ ID No. 2. A nucleic acid according to the invention can be amplified by using cDNA, or alternatively by using genomic DNA as a template, as well as suitable oligonucleotide primers by means of standard PCR amplification techniques. The nucleic acid amplified in this way can be cloned in a suitable vector and characterized by means of DNA sequence analysis. Oligonucleotides corresponding to a LOX nucleotide sequence can be produced by standard synthesis methods, such as an automatic DNA synthesizer. The LOX activity of the proteins encoded by these nucleic acid sequences can then be determined by the enzyme assays described below in the Examples.

In the context of this invention the term "hybridization under stringent conditions" means that the hybridization is performed *in vitro* under conditions stringent enough to ensure a specific hybridization. Stringent *in vitro* hybridization conditions are known to the person skilled in the art, and can be found in the literature (e.g. Sambrook and Russell (2001) Molecular Cloning: A Laboratory Manual, 3rd edition, Cold Spring Harbour Laboratory Press, Cold Spring Harbour, NY). The term "specific hybridization" refers to the fact that a molecule preferably binds to a certain nucleic acid sequence, the target sequence, under stringent conditions, if the target sequence is part of a complex mixture of, for example, DNA or RNA molecules, but does not bind, or at least to a considerably lesser degree, to other sequences.

Stringent conditions depend on the circumstances. Longer sequences hybridize specifically at higher temperatures. In general, stringent conditions are selected so that the hybridization temperature is approximately 5°C below the melting point (Tₘ) for the specific sequence at a defined ionic strength and a defined pH value. Tₘ is the temperature (at a defined pH value, a defined ionic strength and a defined nucleic acid concentration) at which 50% of the molecules complementary to the target sequence hybridize to the target sequence in the equilibrium state. Typically, stringent conditions are those in which the salt concentration is at least about 0.01 to 1.0 M of sodium ion concentration (or the concentration of another salt) at a pH of between 7.0 and 8.3 and the temperature is at least 30°C for short molecules (i.e. for example 10 to 50 nucleotides). Furthermore, stringent conditions can comprise the addition of agents, such as formamide, which destabilizes the hybrids. In hybridization under stringent conditions as used herein, nucleotide sequences which are at least 60% homologous to each other usually remain hybridized to each other. Preferably, the stringent conditions are selected in such a way that sequences which are homologous to each other by at least about 65%, preferably at least about 70%, and especially preferably at least about 75%, or more, usually remain hybridized to each other. A preferred, non-limiting example for stringent hybridization conditions are hybridizations in 6 x sodium chloride/sodium citrate (SSC) at about 45°C, followed by one or more washing steps in 0.2 x SSC, 0.1% SDS at 50 to 65°C. The temperature ranges, for example, under standard hybridization conditions depending on the type of nucleic acid, between 42°C and 58°C in an aqueous buffer at a concentration of 0.1 to 5 x SSC (pH7.2).

If an organic solvent, e.g. 50% formamide, is present in the above-mentioned buffer, the temperature under standard conditions is about 42°C. Preferably, the hybridization conditions for DNA:DNA hybrids are for example 0.1 x SSC and 20°C to 45°C, preferably 30°C to 45°C. Preferably, the hybridization conditions for DNA:RNA hybrids are for example 0.1 x SSC and 30°C to 55°C, preferably between 45°C to 55°C. The hybridization temperatures mentioned above are determined for example for a nucleic acid having a length of about 100 base pairs and a G/C content of 50% in the absence of formamide. The person skilled in the art knows how the required hybridization conditions can be determined using the above mentioned, or the following, textbooks: Current Protocols in Molecular Biology, John Wiley & Sons, N.Y. (1989), Hames und Higgins (publisher) 1985, Nucleic Acids Hybridization: A Practical Approach, IRL Press at Oxford University Press, Oxford; Brown (publisher) 1991, Essential Molecular Biology: A Practical Approach, IRL Press at Oxford University Press, Oxford.

The term "sequence identity" in terms of the invention denotes identity, i.e. the same nucleotides in the same 5'-3' sequence, across the entire sequence of the nucleic acid sequence depicted in SEQ ID No. 3 or SEQ ID No. 4, of at least 60%, 70%, 75%, 80%, preferably of at least 85%, especially preferred of at least 90%, 93% and most preferred of at least 95%, 97%, 99%.

The sequence identity is determined by means of a number of programs which are based on various algorithms. The algorithms of Needleman and Wunsch, or of Smith and Waterman, provide particularly reliable results. For the sequence comparisons, the program PileUp (Feng and Doolittle (1987) J. Mol. Evolution 25: 351 - 360; Higgins et al. (1989) CABIOS 5: 151 - 153), or the programs Gap and Best Fit (Needleman and Wunsch (1970) J. Mol. Biol. 48: 443 - 453, and Smith and Waterman (1981) Adv. Appl. Math. 2: 482 - 489) were used, which are contained in the GCG software package (Genetics Computer Group, 575 Science Drive, Madison, Wisconsin, USA).

The sequence identity values stated above in percent were determined using the program GAP across the entire sequence region with the following settings: gap weight: 50, length weight: 3, average match: 10,000 and average mismatch: 0.000.

Unless stated otherwise, these settings were used as the standard settings for sequence comparisons.

Nucleic acid sequences deviating from the nucleic acid sequences given in SEQ ID No.3 and SEQ ID No.4 can, for example, be created by the insertion of one or several nucleotide substitutions, additions, or deletions in a nucleotide sequence of SEQ ID No. 3 and SEQ ID No. 4, so that proteins are created into which one or more amino acid substitutions, additions, or deletions were inserted as compared to the sequences stated in SEQ ID No. 1 or SEQ ID No. 2. Mutations can be inserted in one of the sequences of SEQ ID No. 3 and SEQ ID No. 4 using standard techniques, such as site specific mutagenesis and PCR-mediated mutagenesis. Preferably, conservative amino acid substitutions are generated at one or several of the predicted nonessential amino acid residues, that is at amino acid residues which do not influence the enzymatic activity of the LOX. In a "conservative amino acid substitution" an amino acid residue is replaced with an amino acid residue having a similar side chain. Families of amino acid residues having similar side chains have been defined in the area of expertise. These families comprise amino acids having basic side chains (such as lysine, arginine, histidine), acidic side chains (such as aspartic acid and glutamic acid), uncharged polar side chains (such as glycine, asparagine, glutamine, serine, threonine, tyrosine, cysteine), nonpolar side chains (such as alanine, valine, leucine, isoleucine, proline, phenylalanine, methionine, tryptophan), beta-branched side chains (such as threonine, valine, isoleucine) and aromatic side chains (such as tyrosine, phenylalanine, tryptophan). A predicted nonessential amino acid residue in the LOX used according to the invention will thus preferably be replaced by another amino acid residue from the same family of side chains. Alternatively, in another embodiment the mutations may be inserted randomly across the entire sequence or a part of it encoding the LOX, for example, by means of saturation mutagenesis, and the resulting mutants may be screened for the LOX activity by recombinantly expressing the encoded protein, in order to identify mutants which have retained the LOX activity. The LOX activity of the protein can, for example, be determined using the assays described herein.

In terms of the invention, "transgenic" or "recombinant" means, with regard to e.g. a nucleic acid sequence, an expression cassette (= gene construct), or a vector containing the nucleic acid sequence according to the invention, or an organism transformed with the respective nucleic acid sequences, expression cassettes, or vectors, all of those constructs being produced by means of genetic technologies, that either
a) the nucleic acid sequence according to the invention, or
b) a genetic control sequence functionally linked to the nucleic acid sequence according to the invention, such as a promoter, or
c) a) and b)
is not in its natural genetic environment, or has been modified by genetic techniques, the modification being, for example, a substitution, addition, deletion, inversion, or insertion of one or several nucleotide residues. Natural genetic environment means the natural genomic, or chromosomal locus in the parental organism, or the existence in a genomic library. In case of a genomic library, the natural genetic environment of the nucleic acid sequence is preferably at least partially conserved. The environment flanks the nucleic acid sequence at least on one side, and has a sequence length of at least 50 bp, preferably at least 500 bp, especially preferably at least 1000 bp, particularly preferably at least 5000 bp. A naturally occurring expression cassette - such as the naturally occurring combination of the natural promoter of the LOX with the respective LOX genes - becomes a transgenic expression cassette when it is modified by means of non-natural, synthetic ("artificial") methods, such as a mutagenization. Corresponding methods are described in US 5,565,350 or WO 00/15815, for example.

In terms of the invention, the term transgenic plant, or plant cell, or transgenic bacterial cell or transgenic yeast cell means, as described above, that the nucleic acids used in the method are not at their natural site in the genome of the plant, or the plant cell or transgenic bacterial cell or transgenic yeast cell, whereby the nucleic acids can be homologously or heterologously expressed. However, transgenic also means that although the nucleic acids according to the invention are located at their natural site in the genome of an organism, the sequence has been changed compared to the natural sequence, and/or the regulatory sequences of the natural sequences have been modified. Preferably, transgenic is to be understood as the expression of the nucleic acids according to the invention at a non-natural site in the genome, i.e. the nucleic acids are homologously, or preferably heterologously expressed.

It is obvious to the person skilled in the art that the nucleic acid sequence used for the production of the transgenic plant, or plant cell, or transgenic bacterial cell or transgenic yeast cell which encodes a LOX, may have to be adjusted to the organism specific codon usage. The codon usage can be determined with computer analyses of other known genes of the selected organism.

The LOX of the present invention has a specificity for the C13 position within the fatty acid with 18 carbon atoms, i.e. it oxygenates preferably at carbon atom 13 of the C18 hydrocarbon backbone of the fatty acid leading to (13S)-hydroperoxy derivatives of the fatty acid. At least 50% or 60%, preferably at least 70% or 80%, more preferably at least 85% or 90%, even more preferably at least 92, 94 or 96% and most preferably 98 or 99% of the oxygenation reactions catalyzed by the LOX of the present invention occur at carbon atom 13.

Furthermore, the present invention relates to a method further comprising
- recovering the produced polyunsaturated fatty acid hydroperoxides and/or
- using the produced polyunsaturated fatty acid hydroperoxides for the production of flavor ingredients and/or fragrance ingredients and/or
- using the produced polyunsaturated fatty acid hydroperoxides as pesticidal or fungicidal compounds and/or
- using the produced polyunsaturated fatty acid hydroperoxides for the production of pesticidal or fungicidal compounds.

The hydroperoxides may be recovered from the transgenic organism or the *in vitro* reaction mixture by extraction and adsorption techniques which are well-known to the person skilled in the art. The thus recovered hydroperoxides may then be used as pesticidal and/or fungicidal compounds.

If the polyunsaturated fatty acid hydroperoxides are to be used for the production of flavor ingredients and/or fragrance ingredients, they are typically cleaved to the respective aldehydes by a lyase. For example, n-hexanal is produced from the hydroperoxide of LA and 3-(Z)-hexen-1-al is produced from the hydroperoxide of LeA. The 3-(Z)-hexen-1-al can be isomerized to obtain 2-(E)-hexen-1-al. Furthermore, the aldehydes n-hexanal, 3-(Z)-hexen-1-al and 2-(E)-hexen-1-al can be reduced to yield their corresponding alcohols n-hexanol, 3-(Z)-hexen-1-ol and 2-(E)-hexen-1-ol. The expert knows how the cleavage, isomerization and reduction reactions have to be performed. Examples of such reactions are given in US 5,464,761; US 6,780,621 and US 6,200,794. The desired products obtained can be separated from the reaction medium by means of steam distillation and/or extraction with an inert organic solvent.

The fatty acid hydroperoxides are cleaved by the action of the enzyme hydroperoxide lyase into a C-6 aldehyde and a C-12 ω-oxoacid moiety. The hydroperoxide lyase can either be used in isolated form or in an extract, e.g. from guava. Nucleic acid sequences coding for a hydroperoxide lyase have been cloned from different organisms, e.g. from tea leaves (Matsui et al. (1991) Phytochemistry 30: 2109-2113), green bell pepper fruits (Shibata et al. (1995) Plant Cell Physiology 36: 147-156), tomato leaves (Fauconnier et al. (1997) J. Agric. Food Chem. 45: 4232), banana (European patent application EP 0 801 133) and guava (US 6,200,794).

The aldehydes may be reduced to their corresponding alcohols for example by adding fresh baker's yeast to the reaction solution. The yeast contains an active alcohol dehydrogenase enzyme that reduces the aldehydes.

After their insertion into a plant cell, or plant, the nucleic acids used in the method can either be located on a separate plasmid, or advantageously be integrated into the genome of the host cell. In the case of integration into the genome, the integration can occur randomly, or by means of such a recombination that the native gene is replaced by the inserted copy, which causes the modulation of cellular LOX expression, or by using a gene in trans so that the gene is functionally linked to a functional expression unit which contains at least one sequence ensuring the expression of a gene, and at least one sequence ensuring the polyadenylation of a functionally transcribed gene.

In addition to the nucleic acid sequence for the LOX to be transferred, the recombinant nucleic acid molecules which are used for the expression of the LOX comprise further regulatory elements. Which precise regulatory elements these vectors have to contain depends in each case on the process in which these vectors are to be used. The person skilled in the art and familiar with the various methods mentioned above for the production of transgenic plants in which the expression of a protein is increased, knows which regulatory and other elements these vectors must contain.

Typically, the regulatory elements which are part of the vectors are such that allow for the transcription and, if desired, for the translation in the plant cell, bacterial cell or yeast cell. Depending on the organism selected, this can mean, for example, that the gene is only expressed and/or overexpressed after induction, or that it is expressed and/or overexpressed immediately. For example, these regulatory sequences are sequences to which inductors or repressors bind, thereby regulating the expression of the nucleic acid. In addition to these new regulatory sequences, or instead of these sequences, the natural regulation of the sequences upstream of the actual structural genes may still be existent, and may possibly have been genetically modified so that the natural regulation is disabled, and the expression of the genes is increased. The recombinant nucleic acid molecule, however, can also be constructed in a simpler manner, i.e. no additional regulation signals are inserted upstream of the nucleic acid sequence, and the natural promoter with its regulation has not been removed. Instead, the natural regulatory sequence has been mutated in such a manner that regulation no longer occurs, and/or gene expression is increased. To increase the activity, these altered promoters can also be inserted singly, in the form of partial sequences, upstream of the natural gene. Furthermore, the gene construct can also advantageously contain one or more so-called enhancer sequences which are functionally linked to the promoter, and which allow an increased expression of the nucleic acid sequence. Additional useful sequences, such as additional regulatory elements or terminators, can also be inserted at the 3' end of the DNA sequences.

In principle, it is possible to use any of the natural promoters with their regulation sequences for the method according to the invention. However, it is also possible and advantageous to use synthetic promoters only or in addition.

For the expression in plant cells, the promoters can be either constitutive, inducible, tissue-specific or development-specific promoters. The choice of promoter, as well as of other regulatory sequences, determines the regional and temporal expression pattern, and therefore also the LOX expression in transgenic plants.

Constitutive promoters include, for example, the core promoter of the Rsyn7 promoter as well as other constitutive promoters described in WO 99/43838 and US patent No 6,072,050; the CaMV 35S promoter (Odell et al. (1985) Nature 313: 810 - 812); the actin promoter (McElroy et al. (1990) Plant Cell 2:163 - 171); the ubiquitin promoter (Christensen et al. (1989) Plant Mol. Biol. 12: 619 - 632 and Christensen et al. (1992) Plant Mol. Biol. 18: 675 - 689); the pEMU promoter (Last et al. (1991) Theor. Appl. Genet. 81: 581 - 588); the MAS promoter (Velten et al. (1984) EMBO J. 3: 2723 - 2730); the ALS promoter (US application No 08/409,297), and similar promoters. When using a constitutive promoter, a cell-specific or tissue-specific expression can also be achieved by inhibiting the gene expression in the cells, or tissues, in which they are not desired, e.g. by forming antibodies which bind the gene product and thus prevent its activity, or by suitable inhibitors which act in these cells.

Preferably, the LOX gene is expressed under control of an inducible promoter, and especially preferably by a pathogen-inducible promoter. Such promoters include those of pathogen-related proteins (PR proteins) which are induced after the infection with a pathogen, such as PR proteins, SAR proteins, beta-1,3 glucanase, chitinase, etc. (see, for example, Redolfi et al. (1983) Neth. J. Plant Pathol. 89: 245 - 254; Uknes et al. (1992) Plant Cell 4: 645 - 656; and Van Loon (1985) Plant Mol. Virol. 4: 111 - 116).

Also of particular interest are promoters which are expressed locally at or near the site of pathogen infection, such as those described by Marineau et al. (1987) Plant Mol. Biol. 9: 335 - 342; Matton et al. (1989) Molecular Plant-Microbe Interactions 2: 325 - 331; Somsisch et al. (1986) Proc. Natl. Acad. Sci. USA 83: 2427 - 2430; Somsisch et al. (1988) Mol. Gen. Genet. 2: 93 - 98; Yang (1996) Proc. Natl. Acad. Sci. USA 93: 14972 - 14977; Chen et al. (1996) Plant J. 10: 955 - 966; Zhang et al. (1994) Proc. Natl. Acad. Sci. USA 91: 2507 - 2511; Warner et al. (1993) Plant J. 3: 191-201; Siebertz et al. (1989) Plant Cell 1: 961 - 968.

Furthermore, wound-inducible promoters are also preferred for use in the method according to the present invention, since pathogens often enter through wounds. Such wound-inducible promoters include that of the proteinase inhibitor (pin II) of the potato (Ryan (1990) Ann. Rev. Phytopathol. 28: 425 - 449; Duan et al. (1996) Nature Biotechnology 14: 494 - 498); wun1 and wun2 (US-Patent Nr. 5,428,148); win1 and win2 (Stanford et al. (1989) Mol. Gen. Genet. 215: 200 - 208); Systemin (McGurl et al. (1992) Science 225: 1570 - 1573); WIP1 (Rohmeier et al. (1993) Plant Mol. Biol. 22: 783 - 792; Eckelkamp et al. (1993) FEBS Letters 323: 73 - 76); of the MPI gene (Corderok et al. (1994) Plant J. 6 (2): 141 - 150); the FGAM-Synthase (Vaghchhipawala et al. (2004) Genome 47 (2): 404 - 413; prxC2 (Kaothien et al. (2002) Plant Mol. Biol. 49 (6): 591 - 599); poxA (Ito et al. (2000) Plant Science 155 (1): 85 - 100); FAD7 (Nishiuchi et al. (1999) Plant Physiol. 121 (4): 1239 - 1246); TR2' (WO 03/093483).

Chemically regulated promoters can be used in order to regulate the expression of a gene in a plant by the use of an exogenous chemical regulator (see review in Gatz (1997) Annu. Rev. Plant Physiol. Plant Mol. Biol., 48: 89-108). Chemically inducible promoters are particularly suited whenever it is desired that the gene expression occurs in a time specific manner. Examples for this include the In2-2 promoter of corn, which is activated by benzenesulfonamide, the GST promoter of corn, which is induced by hydrophobic electrophilic compounds, and the PR-1a promoter of tobacco, which is activated by salicylic acid. Other chemically regulated promoters include steroid responsive promoters (see, for example, the glucocorticoid-inducible promoter in Schena et al. (1991) Proc. Natl. Acad. Sci. USA 88: 10421 - 10425 and McNellis et al. (1998) Plant J. 14 (2): 247 - 257), ethanol-inducible and tetracycline-inducible promoters (see, for example, Gatz et al. (1991) Mol. Gen. Genet. 227: 229 - 237; US patent Nos. 5,814,618 and 5,789,156).

The person skilled in the art knows that the use of inducible promoters allows the production of plants, or plant cells, which express only transiently the sequences according to the invention. Such transient expression allows the production of plants which only exhibit transient pathogen resistance. Such transient resistance can be desirable, for example, when the risk of a pathogen contamination is looming, and the plants therefore need to be resistant to the pathogen for only a certain period. Additional situations, in which a transient resistance is desirable, are known to the person skilled in the art. Furthermore, the person skilled in the art also knows that transient expression, and thereby transient resistance can be achieved by the use of vectors non-stably replicating in plant cells, and carrying the respective sequences for the expression of the LOX.

Tissue-specific promoters can also be used in order to achieve increased LOX expression within a certain plant tissue. Suitable tissue-specific promoters are, for instance, those allowing leaf-specific expression. These include those described in Yamamoto et al. (1997) Plant J. 1 (2): 255 - 265; Kwon et al. (1994) Plant Physiol 105: 357-367; Yamamoto et al. (1994) Plant Cell Physiol. 35 (5): 773-778; Gotor et al. (1993) Plant J. 3: 509-518; Orozco et al. (1993) Plant Mol. Biol. 23 (6): 1129-1138: Matsuoka et al. (1993) Proc. Natl. Acad. Sci. USA 90 (20): 9586-9590; Stockhaus et al. (1987) Proc. Natl. Acad. G. USA 84: 7943 - 7947; Stockhaus et al. (1989) EMBO J. 8: 2445 - 2451.

The use of epidermis-specific promoters is also particularly preferred, since the task of the epidermis as the outer tissue of the overground organs of higher plants is among other things to prevent pathogens from penetrating into the plant. Suitable epidermis-specific promoters include, among others, the promoter of the CER6 (CUT1) gene of *Arabidopsis* (Hooker et al. (2002) Plant Physiol. 129 (4): 1568 - 1580 and Kunst et al. (2000) Biochem. Soc. Trans. 28 (6): 651 - 654).

Other preferred promoters are those that are especially active in fruit. These include, for example, the promoter of a polygalacturonase gene, e.g. of the tomato (Nicholass et al. (1995) Plant Mol. Biol. 28:423-435), the promoter of an ACC oxidase, e.g. of apple (Atkinson et al. (1998) Plant Mol. Biol. 38:449-460), or the 2A11 promoter of tomato (van Haaren et al. (1991) Plant Mol. Biol. 17:615-630).

Also preferred are mesophyll-specific promoters, such as the rbcs promoter of rice or tomato (Kyozuka et al. (1993) Plant Physiol. 102 (3): 991-1000), the PPCZml promoter of corn (Kausch et al. (2001) Plant Mol Biol. 45 (1): 1-15), the CAB2 promoter of Arabidopsis (Thain et al. (2002) Plant Physiol. 130: 102-110), or the AldP promoter of rice (Kagaya et al. (1995) Mol Gen Genet. 248 (6): 668-674).

Furthermore, the person of average skilled in the art is able to isolate additional suitable promoters using routine methods. Thus, the person skilled in the art using current molecular biological methods, such as hybridization experiments, or DNA protein binding studies is able to identify e.g. additional epidermis-specific regulatory nucleic acid elements. Here, for example, the desired tissue is isolated in a first step from the desired organism from which the regulatory sequences are to be isolated, the entire poly(A)⁺ RNA is isolated from it, and a cDNA library is created. In a second step, using cDNA clones which are based on poly(A)⁺ RNA molecules from another tissue, those clones are identified from the first bank by means of hybridization whose corresponding poly(A)⁺ RNA molecules merely accumulate in the desired tissue. Then, using the cDNAs thus identified, promotors are isolated which have tissue-specific regulatory elements. Additional PCR based methods for the isolation of suitable tissue-specific promoters are also available to the person skilled in the art.

The vectors according to the invention, as the regulatory elements, can additionally comprise, e.g. enhancer elements. They may also contain resistance genes, replication signals, and additional DNA regions, which enable propagation of the vectors in bacteria, such as *E. coli.* The regulatory elements also comprise sequences which effect a stabilization of the vectors in the host cells. Such regulatory elements particularly comprise sequences facilitating stable integration of the vector into the host genome of the plant, or an autonomous replication of the vector in the plant cells. Such regulatory elements are known to the person skilled in the art.

The so-called termination sequences are sequences which ensure the proper termination of transcription, or translation. If the transferred nucleic acids are to be translated, the termination sequences are typically stop codons and respective regulatory sequences; if the transferred nucleic acids are only to be transcribed, they are generally poly(A) sequences.

As used herein, the term "vector" relates to a nucleic acid molecule which can transport another nucleic acid, to which it is bound, into a cell. A vector type is a "plasmid" representing a circular double stranded DNA loop, into which additional DNA segments can be ligated. Another vector type is a viral vector, wherein additional DNA segments can be ligated into the viral genome. Certain vectors can replicate autonomously in a host cell into which they have been inserted (e.g. bacterial vectors with a bacterial replication origin). Other vectors are advantageously integrated into the genome of a host cell when inserted in the host cell, and thereby replicated together with the host genome. Also, certain vectors can control the expression of genes to which they are functionally linked. These vectors are called here "expression vectors." Usually, expression vectors suitable for DNA recombination techniques are of the plasmid type. In the present description "plasmid" and "vector" can be used interchangeably, since the plasmid is the vector type most often used. However, the invention is also intended to comprise other types of expression vectors, such as viral vectors which fulfil similar functions. Furthermore, the term "vector" is also intended to comprise other vectors known to the person skilled in the art, such as phages, viruses, such as SV40, CMV, TMV, transposons, IS elements, phasmids, phagemids, cosmids, linear or circular DNA.

In a recombinant expression vector, the term "operatively linked thereto" means that the nucleotide sequence of interest is linked to the regulatory sequence(s) in such a way that the expression of the nucleotide sequence is possible, and that both sequences are linked to each other in such a way so as to fulfil the predicted function ascribed to the sequence.

The term "regulatory sequence" is intended to comprise promoters, enhancers, and other expression control elements (e.g. polyadenylation signals). These regulation sequences are described e.g. in Goeddel: Gene Expression Technology: Methods in Enzymology 185, Academic Press, San Diego, CA (1990), or in Gruber and Crosby, in: Methods in Plant Molecular Biology and Biotechnology, CRC Press, Boca Raton, Florida, publisher: Glick and Thompson, Chapter 7, 89-108. Regulatory sequences comprise those sequences which regulate the constitutive expression of a nucleotide sequence in many types of host cells, and those sequences which regulate the direct expression of the nucleotide sequence only in certain host cells under certain conditions. The person skilled in the art knows that the design of the expression vector can depend on factors, such as the choice of the host cell to be transformed, the desired extent of the protein expression, etc.

The recombinant expression vectors used for the expression of the LOX can be active in both prokaryotic and eukaryotic cells. This is advantageous, since intermediate steps of the vector construction are often performed for the sake of simplicity in microorganisms. These cloning vectors contain a replication signal for the respective microorganism, and a marker gene for the selection of successfully transformed bacterial cells. Suitable vectors for expression in prokaryotic organisms are known to the person skilled in the art; they include e.g. *E. coli* pEXP5-NT/TOPO, pMAL series, pLG338, pACYC184, the pBR series, such as pBR322, the pUC series, such as pUC18, or pUC19, the M113mp series, pKC30, pRep4, pHS1, pHS2, pPLc236, pMBL24, pLG200, pUR290, pIN-III113-B1, λgt11, or pBdCl, *Streptomyces* pIJ101, pIJ364, pIJ702, or pIJ361, *Bacillus* pUB110, pC194, or pBD214, *Corynebacterium* pSA77, or pAJ667.

In another embodiment the expression vector represents a yeast expression vector or a bacillovirus expression vector.

The above named vectors provide only a small overview of possible suitable vectors. Additional plasmids are known to the person skilled in the art and are described in e.g.: Cloning Vectors (publisher Pouwels, P.H. et al. Elsevier, Amsterdam, New York-Oxford, 1985). For additional suitable expression systems for prokaryotic and eukaryotic cells see chapters 15 and 16 of Sambrook and Russell, vide supra.

In another embodiment of the method, the LOX can be expressed in single-celled plant cells (such as algae), see Falciatore et al., 1999, Marine Biotechnology 1 (3):239-251 and the literature cited therein, and in plant cells of higher plants (e.g. spermatophytes, such as crop plants). Examples for plant expression vectors comprise those extensively described in: Becker, D., Kemper, E., Schell, J., and Masterson, R. (1992), Plant Mol. Biol. 20:1195-1197; and Bevan, M.W. (1984), Nucl. Acids Res. 12:8711-8721; Vectors for Gene Transfer in Higher Plants; in: Transgenic Plants, Bd. 1, Engineering and Utilization, publisher: Kung and R. Wu, Academic Press, 1993, S. 15-38.

Since the expression of plant genes is frequently not limited to the transcription level, a plant expression cassette preferably contains, in addition to the elements described above, other functionally linked sequences such as translation enhancers, e.g. the overdrive sequence containing the 5' untranslated leader sequence of the tobacco mosaic virus, which increases the protein/RNA ratio (Gallie et al. (1987) Nucl. Acids Research 15:8693-8711).

The gene to be expressed must, as described above, be functionally linked to a suitable promoter which regulates the gene expression in a time specific, cell specific or tissue specific manner. Suitable promoters have already been described above.

Other preferred sequences for the use in the functional connection in gene expression cassettes are targeting sequences which are required for the targeting of the gene product into the respective cell compartment (see an overview in Kermode (1996) Crit. Rev. Plant Sci. 15 (4): 285-423 and the literature cited therein), such as into the vacuole, the nucleus, all types of plastids, such as amyloplasts, chloroplasts, chromoplasts, the extracellular space, the mitochondria, the endoplasmatic reticulum, elaiosomes, peroxisomes, and other compartments of plant cells.

In order to insert the LOX sequence into the expression vectors, they are advantageously subjected to an amplification and ligation in the known manner. Preferably, one proceeds in accordance with the protocol of the Pfu DNA polymerase, or a Pfu/Taq DNA polymerase mixture. The primers are selected in accordance with the sequence to be amplified. Advantageously, the primer should be selected so that the amplificate comprises the entire codogenic sequence from the start codon to the stop codon. Advantageously, the amplificate is analyzed subsequent to the amplification. For example, the analysis can be made in respect of quality and quantity after gel electrophoretic separation. The amplificate can then be purified according to a standard protocol (e.g. Qiagen). An aliquot of the purified amplificate is then available for the subsequent cloning.

Suitable cloning vectors are generally known to the person skilled in the art. These particularly include vectors which are replicable in microbial systems, i.e. especially vectors which allow for an efficient cloning in bacteria, yeasts or fungi, and which allow for the stable transformation of plants. Especially worth mentioning are various binary and co-integrated vector systems suitable for the T-DNA mediated transformation. Such vector systems are usually characterized in that they contain at least the vir-genes needed for the agrobacteria mediated transformation, as well as the T-DNA limiting sequences (T-DNA border). Preferably, these vector systems also comprise further cis regulatory regions, such as promoters and terminators and/or selection markers used to identify the respective transformed organisms. While vir genes and T-DNA sequences are arranged on the same vector in co-integrated vector systems, binary systems are based on at least two vectors, one of which carries vir genes, but no T-DNA, and a second carries T-DNA, but no vir genes. The latter vectors are thus relatively small, easy to manipulate and replicable both in *E. coli* as well as in agrobacterium. These binary vectors include vectors of the series pBIB-HYG, pPZP, pBecks, pGreen. According to the invention, Bin19, pBI101, pBinAR, pGPTV and pCAMBIA are preferred. An overview of binary vectors and their use is provided by Hellens et al. (2000) Trends in Plant Science 5, 446-451.

For the preparation of the vector, the vectors can initially be linearized by means of restriction endonuclease(s) and then enzymatically modified in any suitable way. The vector is then purified and an aliquot is used for cloning. During cloning the enzymatically cut and if necessary purified amplificate is linked to similarly prepared vector fragments by means of a ligase. A certain nucleic acid construct, or vector construct, or plasmid construct, may have one, or even several, codogenic gene regions. Preferably, the codogenic gene regions in these constructs are functionally linked to regulatory sequences. The regulatory sequences especially include plant sequences, such as the promoters and terminators described above.

The constructs can be advantageously cultivated in microorganisms, especially in *E. coli* and *Agrobacterium tumefaciens,* in a suitable medium, and stably propagated under selection conditions. The cells are then harvested and lysed and the plasmid is extracted therefrom. This allows a transfer of heterologous DNA into plants or microorganisms.

With the advantageous use of cloning vectors, the nucleic acids used in the method according to the invention, the nucleic acids and the nucleic acid constructs according to the invention can be inserted into organisms, such as microorganisms, or preferably plants, and used for plant transformation, just as those published and cited in: Plant Molecular Biology and Biotechnology (CRC Press, Boca Raton, Florida), Chapters 6/7, p. 71-119 (1993); F.F. White, Vectors for Gene Transfer in Higher Plants; in: Transgenic Plants, Bd. 1, Engineering and Utilization, publisher: Kung and R. Wu, Academic Press, 1993, 15-38; B. Jenes et al., Techniques for Gene Transfer, in: Transgenic Plants, Bd. 1, Engineering and Utilization, publisher: Kung and R. Wu, Academic Press (1993), 128-143; Potrykus (1991) Annu. Rev. Plant Physiol. Plant Molec. Biol. 42: 205-225. The nucleic acids used in the method, the nucleic acids and nucleic acid constructs, and/or vectors according to the invention, can therefore be used for the genetic modification of a broad spectrum of organisms, preferably of plants.

There are plurality of known techniques available for inserting DNA into a plant host cell, and the person skilled in the art will have no difficulty in finding the most suitable method in each case. These techniques comprise the transformation of plant cells with T-DNA using *Agrobacterium tumefaciens* or *Agrobacterium rhizogenes* as transformation agent, the fusion of protoplasts, the direct gene transfer of isolated DNA into protoplasts, the electroporation of DNA, the insertion of DNA by means of the biolistic method, as well as other possibilities. Both stable and transient transformants can be generated in this manner.

For injection and electroporation of DNA in plant cells there are no special demands made per se on the plasmids used. Similar is true for the direct gene transfer. Simple plasmids such as pUC derivatives can be used. However, if whole plants are to be regenerated from such transformed cells, the presence of a selectable marker gene is necessary. The person skilled in the art knows the most commonly used selection markers, and selecting a suitable marker does not present a problem to him. Commonly used selection markers are those which make the transformed plant cells resistent to a biocide or to an antibiotic, such as kanamycin, G418, bleomycin, hygromycin, methotrexate, glyphosate, streptomycin, sulfonylurea, gentamycin, or phosphinotricin the the like. The individually selected marker should allow the selection of transformed cells as opposed to cells lacking the inserted DNA. For this purpose, alternative markers, such as nutrition markers or screening markers (such as GFP, green fluorescent protein) are also suitable. Of course, selection markers may also be completely dispensed with, which however, greatly increases the need for screening. If marker-free transgenic plants are desired, strategies are also available to the person skilled in the art which allow subsequent removal of the marker gene, such as the cotransformation, or sequence-specific recombinases. Once the inserted DNA has been integrated into the genome of the plant cell, it is usually stable there, and will also remain in the progeny of the originally transformed cell.

If agrobacteria are used for the transformations, the DNA to be inserted must be cloned into special plasmids, as explained above, either in an intermediary or in a binary vector. The intermediary vectors can, due to sequences which are homologous to the sequences in the T-DNA, be integrated in the Ti or Ri plasmid of the agrobacteria by means of homologous recombination. The plasmid also contains the vir region necessary for the transfer of the T-DNA. Intermediary vectors are unable to replicate in agrobacteria. The intermediary vector can be transferred to Agrobacterium tumefaciens by means of a helper plasmid (conjugation). Binary vectors can replicate both in E. coli, as well as in agrobacteria. They contain a selection marker gene and a linker or polylinker, which are framed by the right and the left T-DNA boundary region. They can be transformed into the agrobacteria directly (Holsters et al. (1978), Molecular and General Genetics 163, 181-187). The agrobacterium serving as the host cell should contain a plasmid carrying a vir region. The vir region is necessary for the transfer of the T-DNA into the plant cell. T-DNA may also be present. The agrobacterium transformed in this way is used for the transformation of plant cells.

The use of T-DNA for the transformation of plant cells has been extensively analyzed, and sufficiently described in EP 120 515.

For the transfer of the DNA into the plant cell, plant explants can advantageously be cultivated with Agrobacterium tumefaciens or Agrobacterium rhizogenes. Whole plants can then be regenerated from the infected plant material (such as leaf sections, stalk segments, roots, but also protoplasts or suspension-cultivated plant cells) in a suitable medium, which may contain antibiotics or biozides for the selection of transformed cells. The regeneration of the plants is performed according to conventional regeneration methods using known culture media. The plants, or plant cells, obtained in this manner can then be analyzed for the presence of inserted DNA by means of established methods, such as Southern Blot or PCR.

Other possibilities for inserting foreign DNA using the biolistic method or by protoplast transformation are known to the person skilled in the art (see L. Willmitzer (1993) Transgenic Plants in: Biotechnology, A Multi-Volume Comprehensive Treatise (publisher: H.J. Rehm et al.), Volume 2, 627-659, VCH Weinheim, Germany).

Meanwhile not only has the transformation of dicotyledonous plants, or their cells, become well established via Ti-plasmid vector systems by means of Agrobacterium tumefaciens, but also the one of monocotyledonous plants, or their cells (see, among others, Chan et al. (1993), Plant Mol. Biol. 22, 491-506).

Alternative systems for the transformation of monocotyledonous plants, or their cells, include the transformation by means of the biolistic approach (Wan and Lemaux (1994) Plant Physiol. 104, 37-48; Vasil et al. (1993) Bio/Technology 11, 1553-1558; Ritala et al. (1994) Plant Mol. Biol. 24, 317-325; Spencer et al. (1990), Theor. Appl. Genet. 79, 625-631), protoplast transformation, electroporation of partially permeabilized cells, as well as the insertion of DNA by means of fiber glass.

The transformed cells grow within the plant in the usual way (also see McCormick et al. (1986), Plant Cell Reports 5, 81-84). The resulting plants can be cultivated normally, and interbred with plants having the same transformed genetic code, or a different genetic code. The resulting hybrid individuals possess the corresponding phenotypical characteristics.

Two or more generations should be cultivated in order to ensure that the phenotypical characteristic is stably retained and transmitted. Also, seeds should be harvested in order to ensure that the respective phenotype, or other characteristic have been retained.

Likewise, transgenic lines can be identified according to conventional methods which lines are homozygous for the new nucleic acid molecules, and their phenotypical behavior with regard to a present, or absent pathogen responsiveness can be analyzed and compared to the behaviour of hemizygous lines.

Of course, the plant cells containing the nucleic acid molecules according to the invention may also be further cultivated in the form of a cell culture (including protoplasts, calli, suspension cultures, and the like).

According to the invention, the term transgenic plant comprises the plant in its entirety, as well as all parts of the plant in which the expression of the LOX proteins according to the invention is increased. This includes all parts of the plant and plant organs, such as leaf, stem, seed, root, tubers, anthers, fibers, root hair, stalk, embryos, calli, cotelydons, petioles, crop material, plant tissue, reproductive tissue, cell cultures derived from the transgenic plant, and/or which can be used to produce the transgenic plant.

The plants used for the method according to the invention can in principle be any plant which is to be made resistant to a pathogen infestation. Preferably, it is a monocotyledonous or dicotyledonous agricultural plant, a food plant or a fodder plant.

Examples pf monocotyledonous plants are plants belonging to the genera Avena (oat), Triticum (wheat), Secale (rye), Hordeum (barley), Oryza (rice), Panicum, Pennisetum, Setaria, Sorghum (millet), Zea (corn), and the like.

Dicotyledonous agricultural plants comprise, inter alia, cotton, leguminous plants such as pulses, and especially alfalfa, soy bean, oilseed rape. canola, tomato, sugar beet, potato, sunflower, ornamental plants as well as trees. Additional agricultural plants can comprise fruit (especially apples, pears, cherries, grapes, citrus, pineapples, and bananas), oil palms, tea, cocoa and coffee bushes, tobacco, sisal as well as in medical plants Rauwolfia and Digitalis. Especially preferred are the cereals wheat, rye, oats, barley, rice, corn, and millet, as well as the dicotyledonous plants sugar beet, oilseed rape, soy, tomato, potato, and tobacco. Additional agricultural plants can be gathered from US patent No. 6,137,030.

Preferred plants are marigold, sunflower, Arabidopsis, tobacco, red pepper, soy, tomato, eggplant, peppers, carrot, potato, corn, lettuce and types of cabbage, cereals, alfalfa, oats, barley, rye, wheat, triticale, millet, rice, alfalfa, flax, cotton, hemp, Brassicaceae, such as oilseed rape or canola, sugar beet, sugarcane, nut and wine species, or trees, such as aspen or yew tree.

Depending on the vector system used, transgenic plants can also be generated according to the invention, in which the nucleic acids to be transferred are contained in the plant cell, or the plant, as an independently replicating system. The vectors used for the transfer of the plants must then possess the corresponding DNA sequences which facilitate the replication of the plasmids used for the transfer within the cell.

The specific expression of the LOX protein in the plants, or plant cells or bacterial cells or yeast cells, according to the invention can be proven and tracked by means of common molecular biological and biochemical methods. The person skilled in the art knows these techniques and is easily able to select suitable detection methods, such as a Northern Blot analysis for the detection of LOX-specific RNA, or for the determination of the amount of accumulation of LOX-specific RNA, or a Southern Blot, or PCR, analysis for the detection of DNA sequences encoding the LOX. The probe or primer sequences used for this purpose can either be identical to the sequences given in SEQ ID No. 3 or 4, or show some slight differences to these sequences.

Further preferred host organisms for use in the method according to the present invention are fungi such as for example *Mortierella, Saprolegnia* or *Pythium,* bacteria such as the ones of the genus *Escherichia,* yeasts such as *Saccharomyces,* cyanobacteria, ciliates, algae or protozoa such as dinoflagellates such as *Crypthecodinium.* Industrially used suitable microorganisms include, but are not limited to Gram negative bacteria such as *E. coli,* Gram positive bacteria such *B. subtilis,* fungi such as *A. niger, A. nidulans, N. crassa;* yeasts such as *S. cerevisiae, K. lactis, H. polymorpha, P. pastoris, Y. lipolytica;* actinomycetes such as *Streptomyces* sp. Several of these microorganisms are described in the 'Manual of Industrial Microbiology and Biotechnology' (editors in chief: A. L. Demain, J.E. Davies; ASM Press).

Further suitable host cells can be derived from: Goeddel, Gene Expression Technology: Methods in Enzymology 185, Academic Press, San Diego, CA (1990).

Suitable expression strains, e.g. with a lower protease activity are described in: Gottesman, S., Gene Expression Technology: Methods in Enzymology 185, Academic Press, San Diego, California (1990) 119-128.

If microorganisms such as yeasts such as *Saccharomyces* or *Schizosaccharomyces,* fungi such as *Mortierella, Aspergillus, Phytophtora, Entomophthora, Mucor* or *Traustochytrium,* algae such as *Isochrysis, Phaeodactylum, Chlamydomonas, Volvox* or *Crypthecodinium* are used in the method according to the present invention, these organisms are preferably grown under standard conditions in a fermentation process in manner known to the expert.

The term "standard conditions" refers to the cultivation of a microorganism in a standard medium which is not enriched in serine. The temperature, pH and incubation time can vary as described below.

The standard culture conditions for each microorganism used can be taken from the textbooks, such as Sambrook and Russell, Molecular Cloning - A laboratory manual, Cold Spring Harbour Laboratory Press, 3rd edition (2001).

E.g., *E. coli* and *C. glutamicum* strains are routinely grown in MB or LB and BHI broth (Follettie, M. T. et al. (1993) J. Bacteriol. 175: 4096-4103, Difco Becton Dickinson). Usual standard minimal media for E. coli are M9 and modified MCGC (Yoshihama et al. (1985) J. Bacteriol. 162: 591-507; Liebl et al. (1989) Appl. Microbiol. Biotechnol. 32: 205-210.). Other suitable standard media for the cultivation of bacteria include NZCYM, SOB, TB, CG12 ½ and YT.

"Standard media" within the meaning of the present invention are intended to include all media which are suitable for the cultivation of the microorganisms of the present invention. Both enriched and minimal media are comprised with minimal media being preferred.

Standard media within the context of the present invention do not comprise media to which one or more amino acids have been added or are added.

"Minimal media" are media that contain only the minimal necessities for the growth of wild-type cells, i.e. inorganic salts, a carbon source and water.

In contrast, "enriched media" are designed to fulfil all growth requirements of a specific microorganism, i.e. in addition to the contents of the minimal media they contain for example growth factors.

Antibiotics may be added to the standard media in the following amounts (micrograms per milliliter): ampicillin, 50; kanamycin, 25; nalidixic acid, 25 to allow for the selection of transformed strains.

Suitable media consist of one or more carbon sources, nitrogen sources, inorganic salts, vitamins and trace elements. Preferred carbon sources are sugars, such as mono-, di-, or polysaccharides. For example, glucose, fructose, mannose, galactose, ribose, sorbose, lactose, maltose, sucrose, raffinose, starch or cellulose may serve as very good carbon sources.

It is also possible to supply sugar to the media via complex compounds such as molasses or other by-products from sugar refinement. It can also be advantageous to supply mixtures of different carbon sources. Other possible carbon sources are alcohols and organic acids, such as methanol, ethanol, acetic acid or lactic acid. Nitrogen sources are usually organic or inorganic nitrogen compounds, or materials which contain these compounds. Exemplary nitrogen sources include ammonia gas or ammonia salts, such as NH₄Cl or (NH₄)₂SO₄, NH₄OH, nitrates, urea, amino acids or complex nitrogen sources like corn steep liquor, soy bean flour, soy bean protein, yeast extract, meat extract and others.

Inorganic salt compounds which may be included in the media include the chloride, phosphate or sulfate salts of calcium, magnesium, sodium, cobalt, molybdenum, potassium, manganese, zinc, copper and iron. Chelating compounds can be added to the medium to keep the metal ions in solution. Particularly useful chelating compounds include dihydroxyphenols, like catechol or protocatechuate, or organic acids, such as citric acid. It is typical for the media to also contain other growth factors, such as vitamins or growth promoters, examples of which include biotin, riboflavin, thiamin, folic acid, nicotinic acid, pantothenate and pyridoxin. Growth factors and salts frequently originate from complex media components such as yeast extract, molasses, corn steep liquor and others. The exact composition of the media compounds depends strongly on the immediate experiment and is individually decided for each specific case. Information about media optimization is available in the textbook "Applied Microbiol. Physiology, A Practical Approach (eds. P. M. Rhodes, P.F. Stanbury, IRL Press (1997) pp. 53-73, ISBN 0 19 963577 3). It is also possible to select growth media from commercial suppliers, like standard 1 (Merck) or BHI (brain heart infusion, DIFCO) or others.

All medium components should be sterilized, either by heat (20 minutes at 1.5 bar and 121°C) or by sterile filtration. The components can either be sterilized together or, if necessary, separately.

The preparation of standard media used for the cultivation of bacteria usually does not involve the addition of single amino acids. Instead, in enriched media for use under standard culture conditions a mixture of amino acids such as peptone or trypton is added.

The amount of the protein may be increased by expression of an exogenous version of the respective protein. Further, expression of the endogenous protein can be increased by influencing the activity of the promoter and/or enhancer elements and/or other regulatory activities such as phosphorylation, isoprenylation etc. that regulate the activities of the respective proteins either on a transcriptional, translational or post-translational level.

Besides simply increasing the amount of one or more proteins, the activity of the proteins may be increased by using enzymes which carry specific mutations that allow for an increased activity of the enzyme. Such mutations may, e.g. inactivate the regions of an enzyme that are responsible for feedback inhibition. By mutating these by e.g. introducing non-conservative mutations, the enzyme does not provide for feedback regulation anymore and thus the activity of the enzyme is not down-regulated if more product molecules are produced. The mutations may be either introduced into the endogenous copy of the enzyme, or may be provided by over-expressing a corresponding mutant form of the exogenous enzyme. Such mutations may comprise point mutations, deletions or insertions. Point mutations may be conservative (replacement of an amino acid with a biochemically similar one) or non-conservative (replacement of an amino acid with another which is not biochemically similar). Furthermore, deletions may comprise only two or three amino acids up to complete domains of the respective protein.

Expression of proteins in prokaryotes is most often carried out with vectors containing constitutive or inducible promoters directing the expression of either fusion or non-fusion proteins.

Fusion vectors add a number of amino acids to a protein encoded by the inserted nucleic acid sequence, usually to the amino terminus of the recombinant protein but also to the C-terminus or fused within suitable regions in the proteins. Such fusion vectors typically serve three purposes: 1) to increase expression of recombinant protein; 2) to increase the solubility of the recombinant protein; and 3) to aid in the purification of the recombinant protein by providing a ligand for affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase.

Typical fusion expression vectors include pQE (Qiagen), pGEX (Pharmacia Biotech Inc; Smith, D. B. and Johnson, K. S. (1988) Gene 67: 31-40), pMAL (New England Biolabs, Beverly, MA) and pRIT5 (Pharmacia, Piscataway, NJ) which fuse glutathione S-transferase (GST), maltose E binding protein, or protein A, respectively.

Examples for *C. glutamicum* vectors can be found in the Handbook of Corynebacterium 2005 Eggeling, L. Bott, M., eds., CRC press USA.

Examples of suitable inducible non-fusion *E. coli* expression vectors include pTrc (Amann et al. (1988) Gene 69: 301-315), pLG338, pACYC184, pBR322,pUC18, pUC19, pKC30, pRep4,pHS1, pHS2, pPLc236, pMBL24, pLG200, pUR290,pIN-III113-B1, egtll, pBdCl, and pET lld (Studier et al., Gene Expression Technology: Methods in Enzymology 185, Academic Press, San Diego, California (1990) 60-89; Pouwels et al., eds. (1985) Cloning Vectors. Elsevier: New York ISBN 0 444 904018). Target gene expression from the pTrc vector relies on host RNA polymerase transcription from a hybrid trp-lac fusion promoter. Target gene expression from the pET 11d vector relies on transcription from a T7 gnlO-lac fusion promoter mediated by a coexpressed viral RNA polymerase (T7gnl). This viral polymerase is supplied by host strains BL21 (DE3) or HMS174 (DE3) from a resident X prophage harboring a T7 gnl gene under the transcriptional control of the lacUV 5 promoter. For transformation of other varieties of bacteria, appropriate vectors may be selected. For example, the plasmids pIJ101, pIJ364, pIJ702 and pIJ361 are known to be useful in transforming *Streptomyces,* while plasmids pUB110, pC194, or pBD214 are suitable for transformation of *Bacillus* species. Several plasmids of use in the transfer of genetic information into Corynebacterium include pHM1519, pBL1, pSA77, or pAJ667 (Pouwels et al., eds. (1985) Cloning Vectors. Elsevier: New York IBSN 0 444 904018).

In another embodiment, the protein expression vector is a yeast expression vector. Examples of vectors for expression in yeast (*S. cerevisiae*) include pYepSecl (Baldari, et al. (1987) Embo J. 6: 229-234), 2i, pAG-1, Yep6, Yep13, pEMBLYe23, pMFa (Kurjan and Herskowitz (1982) Cell 30: 933-943), pJRY88 (Schultz et al. (1987) Gene 54: 113-123), and pYES2 (Invitrogen Corporation, San Diego, CA). Vectors and methods for the construction of vectors appropriate for use in other fungi, such as the filamentous fungi, include those detailed in: van den Hondel, C. A. M. J. J. & Punt, P. J. (1991) in: Applied Molecular Genetics of Fungi, J. F. Peberdy, et al., eds., p. 1-28, Cambridge University Press: Cambridge, and Pouwels et al., eds. (1985) Cloning Vectors. Elsevier: New York (ISBN 0 444 904018).

Besides using transgenic organisms or cells such as bacterial, yeast or plant cells and fungi or algae, the 13-LOX enzymes of the present invention may also be expressed in a cell-free system using a suitable expression vector. The cell free expression is a coupled transcription and translation reaction to produce active recombinant protein in high amounts *in vitro* and can be obtained commercially from companies such as Invitrogen (Expressway^{™} Mini Cell-Free Expression System). One general example for such a cell-free expression system is given below.

Furthermore, the invention relates to the use of the polyunsaturated fatty acid hydroperoxides produced according to the invention as pesticidal or fungicidal compounds in controlling plant diseases.

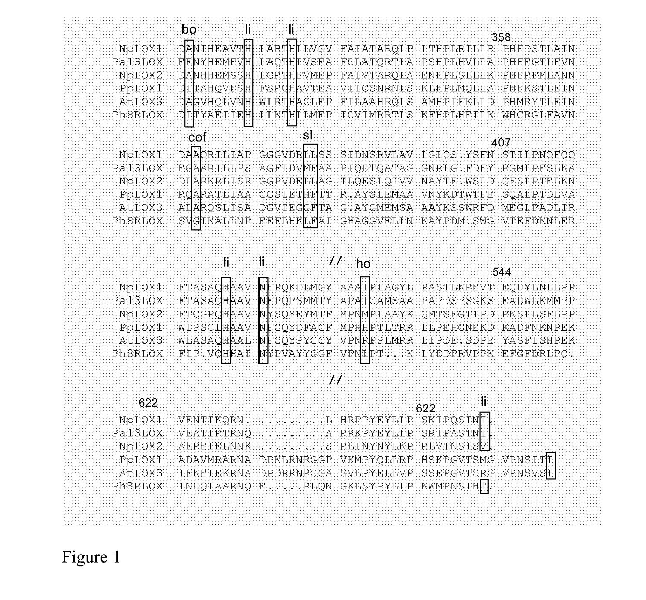

The cyanobacterial 13-LOX enzymes described herein differ from known LOX proteins from other organisms, inter alia, in that they harbour two leucine residues (Leu-384 and Leu-385) at the "Sloane site" (Sloane et al. (1991) Nature 354, 149-152) instead of methionine (Met-667) and phenyalanine (Phe-668) in *PaLOX13* or glycine (His-667) and phenylalanine (Phe-668) in *AtLOX3* (Fig. 1, sl). These amino acids differ remarkably from the determinants described to be involved in determining the positional specificity of plant LOXs, making it impossible to predict the possible positional specificity of both LOXs.

Therefore, in a preferred embodiment the prokaryotic 13-LOX according to the invention is characterized in that it harbours two leucine residues at the so-called Sloane site.

Another feature of a preferred prokaryotic 13-LOX is a missing Arg residue in comparison to most plant LOX enzymes of the prior art which contain said Arg residue as determinant involved in inverse substrate orientation (so-called Hornung determinant; Hornung et al. (1999) Proc. Natl. Acad. Sci. USA 96, 4192-4197). In plant LOXs this Arg residue is localized in the vicinity of the putative substrate-binding pocket and may interact with the carboxy group of the substrates when there is an inverse head to tail substrate orientation (Fig. 1, ho; Fig. 6a). In the LOX enzymes according to the invention this Arg residue is absent.

Further, the 13-LOX enzymes according to the present invention differ from known LOX proteins in that they are smaller and thus easier to handle with respect to their preparation, isolation as well as their use in biochemical reactions to produce 13-LOX-derived hydroxides ofPUFAs, especially of C I 8-PUFAs. While plant LOXs normally have a length of more than 860 amino acids, NpLOX1 and NpLOX2 encode for proteins of 630 and 548 amino acids, respectively.

Accordingly, preferably the 13-LOXs of the invention are proteins of 650 or less amino acids, more preferably of 640 or less amino acids and most preferably of 630 or less amino acids. The molecular weight is preferably less than 75 kDa, more preferably less than 73 kDa and most preferably less than 72 kDa.

Most important, in contrast to prokaryotic LOX enzymes described in the prior art, the cyanobacterial 13-LOX according to the invention is a specific 13-LOX, and not an unspecific linoleate 9/13-LOX.

The identification of NpLOX1 and NpLOX2 from *Nostoc punctiforme* as genes coding for a C13-specific LOX and their characterization will be described below. The following examples should not be construed as limiting. The content of all literature, patent applications, patents, and published patent applications cited in this patent application is incorporated herein by reference.

### EXAMPLES

### 1. Algae Material

*Nostoc punctiforme* PCC 73102 was cultivated in shaking batch cultures at 120 rpm using 11 conical flasks at 20 °C in BG 11 medium for cyanobacteria according to *Sammlung für Algenkulturen in Goettingen* (http://www.epsag.uni-goettingen.de/html/sag.html). Cultures were harvested via centrifugation at OD₆₀₀ 1.2.

### 2. Isolation of the LOXs

Genomic DNA of *Nostoc punctiforme* was isolated from approximately 50 mg of frozen cell material using the NucleoSpin^{®} Plant kit (Macherey-Nagel, Düren, Germany). The genes encoding two LOX proteins were isolated by PCR using the following primers:
*NpLOX40F* (SEQ ID NO: 5):
   5'-ATGACTGCTTTATCACCAGATCATTCAATCAGTTCA'-3;
*NpLOX40R* (SEQ ID NO: 6):
   5'-TCAGATATTGATGCTCTGAGGAATTTTA-3'; and
*NpLOX50F* (SEQ ID NO: 7):
   5'-ATGAAACCATACCTCCCTCAGAATGAT-3';
*NpLOX50R* (SEQ ID NO: 8):
   5'-TCACACGCTAATACTATTTGACACAAG-3'.

The obtained fragments, named LOX40 and LOX50, were cloned into expression vector pEXP5-NT/TOPO^{®} (Invitrogen, Karlsruhe, Germany) with N-terminal His-Tag and transformed into *E.coli* BL21(DE3) cells.

For the cloning reaction the following procedure was performed (see also user manual of Invitrogen)
→ 1 µl of fresh PCR-product
→ 1 µl Salt Solution
→ 1 µ TOPO-vector
→ 3 µl Water

The reaction mix was incubated for 10 min at RT. The reaction was then placed on ice and the transformation of OneShot TOP10 Competent *E.coli* cells could be performed. 2µl of the ligation reaction was added to 50µl competent cells and the reaction was incubated on ice for 20 min. The cells were heat-shocked for 30 seconds at 42°C and immediately transferred back on ice. 250µl S.O.C medium was added ant the tube was shaken horizontally (200 rpm) at 37°C for 1 hour. 10-50 µl from the transformation was spread on a prewarmed selective plate and incubated overnight at 37°C

Next day 10 colonies were picked for analysis. The transformants could either be analyzed by PCR with primers, which had bindings site flanking the gene of interest or by restriction enzymes cutting the plasmid into specific fragments to distinguish between positive and negative clones.

Once the correct clone was obtained the purified plasmid DNA of the expression construct was used for the transformation of the bacterial cell lines BL21, BL21*, Rosetta, HMS and RP.

The transformation was performed as described above. An appropriate transformant was selected and a culture in LB medium containing 100 µg/ml carbenicillin was initiated. The culture was grown at 37°C with shaking until the OD600 was reached 0.6. The culture was induced with 1M IPTG (1:1000) and grown with shaking over night at 16°C.

Furthermore, a pMAL expression vector expressing NpLOX1 or NpLOX2, respectively, was produced as follows. The NpLOX1/2 genes were amplified using the following primers:
NpLOX1F 5'-CGC GGA TCC ATG ACT GCT TTA TCA CCA GAT CAT T-3' (SEQ ID No: 9)
   (with BamHI recognition site)
NpLOX1R 5'-TGCACTGCAGTCAGATATTGATGCTCTGAGGAAT-3' (SEQ ID No: 10)
   (with PstI recognition site)
NpLOX2a 5'-CGGATCCATGAAACCATACCTCCCTCAGAATG-3' (SEQ ID No: 11)
   (with BamHI recognition site)
NpLOX2b 5'-GCTGCAGTTACACGCTAATACTATTAGTCACAAG-3' (SEQ ID No: 12)
   (with PstI recognition site)

The purified PCR-fragments were first ligated into pGEM-T-cloning vector (Promega, Mannheim, Germany). The ligation reaction was transformed into XL1Blue *E. coli* cells and positive clone selected via Blue-white screening (see user manual). The inserted PCR fragment was cut out with specific restriction enzymes (BamHI and PstI). The same enzymes were used to cut the expression vector pMAL-c2x (NewEnglandBiolabs, U.S.), to produce in both, the fragment and the vector, sticky ends. The LOX-fragment was ligated into pMAL-c2x and the prodedure was followed as described for pEXP5-NT cloning strategy.

### 3. Expression analysis of the NpLOX2

The expression vector pEXP5-NT containing the NpLOX2 was transformed into different bacterial strains as described in Example 2. The culture was induced with 1M IPTG (1:1000) and half of the cells was stressed by adding 6% ethanol to the culture medium. After culturing overnight at 16°C, the cells were harvested by centrifugation (4000 rpm, 4°C, 20 min) and sonificated in 2 ml lysis buffer (50 mM Tris/HCl pH 8,0, 150 mM NaCl, 10% glycerol, 0.1% Tween 20). Both the pellet (membrane fraction) and the supernatant were then separated on a 10% SDS-PAGE and then analysed by Coomassie staining (40% (v/v) methanol, 10% (v/v) acetic acid, 0.25% (w/v) Coomassie Brillant Blue 250).

### 4. LOX activity assay

For product identification and analysis of LOX proteins 0.5 ml of *E. coli* cell lysate was extracted with 1.5 ml of 50 mM Tris/HCl (pH 8.0) and incubated with 250 µg of fatty acid substrate (LA, LeA, GLA and AA) on ice. Hydroperoxides were reduced to their corresponding hydroxides with SnCl₂. After acidification to pH 3.0 with glacial acid, the fatty acids were extracted as described (Bligh and Dyer (1959) Can. J. Biochem. Physiol. 37:911-917).

After removing the organic solvent, the residues were reconstituted in 80 µl of methanol/water/acetic acid (85:15:0.1, v/v) and subjected to HPLC analysis. HPLC analysis was performed with an Agilent 1100 HPLC system (Waldbronn, Germany) coupled to a diode array detector. Hydroxy fatty acids were separated from fatty acids by reversed phase HPLC (RP-HPLC; EC250/4 Nucleosil 120-5 C18, Macherey-Nagel, Dueren, Germany) eluted with a solvent system of methanol/water/acetic acid (85:15:0.1, v/v) at a flow rate of 0.2 ml/min. Straight phase HPLC (SP-HPLC) of hydroxy fatty acid isomers was carried out on a Zorbax SIL column (250/4.6, 5 µm particle size, Agilent, Waldbronn, Germany) eluted with a solvent system of hexane/2-propanol/acetic acid (100:5:0.1, v/v) at a flow rate of 0.2 ml/min. CP-HPLC of the hydroxy fatty acids was carried out on a Chiralcel OD-H column (2.1 x 150 mm, 5 - µm particle size, Daicel, distributed by VWR, Darmstadt, Germany) with a solvent system of *n*-hexane/2-propanol/acetic acid (100:5:0.1, v/v) and a flow rate of 0.1 ml/min. The absorbance at 234 nm (conjugated diene system of the hydroxy fatty acids) was recorded simultaneously during all chromatographic steps.

### 5. Determination of LOX activity and LOX products

Endogenous LOX activity was determined using crude extracts from *Nostoc punctiforme* PCC 73102. Determination of hydroxy and hydroperoxy fatty acids was performed by HPLC-based and GC (gas chromatography)-based analyses. To about 1.5 g of frozen algal cells 10 ml of extraction medium (*i*-hexane/2-propanol, 3:2 (v/v) with 0.0025 % (w/v) butylated hydroxytoluene) was added and immediately homogenized with a glass potter under a stream of argon on ice for 30 s. As an internal standard, (6*Z*,9*Z*,11*E*,13*S*)-13-hydroxy-6,9,11-octadecatrienoic acid was added. The extract was centrifuged at 4,500 g at 4 °C for 10 min. The clear upper phase was collected, and the pellet was extracted three times with 3 ml each of extraction medium. To the combined organic phases, a 6.7 % (w/v) solution of potassium sulfate was added to a final volume of 47 ml. After vigorous shaking, the upper hexane rich layer was removed. The upper organic phase containing oxylipins was dried under nitrogen and re-dissolved in 80 µl of methanol/water/acetic acid (85:15:0.1, v/v). At first, oxylipins were purified on RP-HPLC, then SP-HPLC was applied to separate the hydro(pero)xy derivatives of LA and LeA according to "3. LOX activity assay", above.

For the analysis of esterified oxylipins and fatty acids, triricinoleate and triheptadecanoate were used as internal standards, and the extraction was performed as described above. After removing of the solvent, 405 µl of a mixture of toluene and methanol (1:2, v/v) and 150 µl of 0.5 mM sodium methoxide were added. After incubation for 20 min, 0.5 ml of 1 M NaCl and 50 µl of HCl (37 %, v/v) were added, and the resulting fatty acid methyl esters and their oxidized derivatives were extracted twice each with 0.75 ml of hexane. The combined organic phases were evaporated to dryness under a stream of nitrogen and dissolved in 200 µl of methanol. An aliquot of 20 µl of this solution was dried under nitrogen and dissolved in 10 µl of acetonitrile for the analysis of the fatty acid methyl esters by GC/FID as described before (Hornung et al. (2002) Eur. J. Biochem. 269:4852-4859). The methyl esters of the oxylipins were analyzed by HPLC according to the analysis of the free oxylipins (Göbel et al. (2003) J. Biol. Chem. 278:52834-52840). Jasmonic acid and 12-oxo phytodienoic acid were determined by GC/MS as described (Stumpe et al. (2005) Phytochemistry 66:781-791).

### 6. Isolation of two putative LOXs from N. punctiforme PCC73102

To isolate LOX genes from *N. punctiforme* PCC73102, gene specific primers were used to amplify the LOX fragments from genomic DNA. The obtained fragment of NpLOX1 had a complete open reading frame of 1892 bp encoding a protein of 630 amino acids with a molecular size of 70.9 kDa. The open reading frame of NpLOX2 had a length of 1644 bp encoding a protein of 548 amino acids with a molecular size of 62.9 kDa.

To gain more information on the biochemical features of NpLOX1 and 2 their protein sequences were aligned with other characterised LOXs from plant, moss, coral and *P. aeruginosa.* Diagnostic differences or similarities within the structure are mainly found around the active site residues of the proteins. The corresponding partial protein alignment is presented in Fig. 1. The central histidine-rich region, including three histidines (His-327, His-332, His-510 in NpLOX1) which constitute the iron binding site, are highly conserved in all six LOX-sequences (Fig. 1, li). Two additional amino acids, asparagine (Asn-514 in NpLOX1) and isoleucine (Ile-630 in NpLOX1) which may be also involved in the iron binding are highly conserved in NpLOX1 (Fig. 1, li). For NpLOX2 a valine (Val-548) can be found at the C-terminus that corresponds to the position of Ile-630 in NpLOX1. At the position of the three determinants that may be involved in substrate- and regiospecificity both NpLOXs contain different amino acids in comparison to the other four LOX sequences. Particularly, each of the two sequences harbour two leucine residues (Leu-667 and Leu-668) at the "Sloane site" instead of methionine (Met-667) and phenyalanine (Phe-668) in *PaLOX13* or glycine (His-667) and phenylalanine (Phe-668) in *AtLOX3* (Fig. 1, sl). These amino acids differ remarkably from the determinants described to be involved in determining the positional specificity of plant LOXs, making it impossible to predict the possible positional specificity of both LOXs. This is furthermore supported by the observation that the Arg residue as determinant involved in inverse substrate orientation in plant LOXs is missing as well (Fig. 1, ho). However, the amino acid residue determining the stereospecificity of LOXs ("Coffa site") is in both sequences an alanine residue (Fig. 1, cof), identifying both LOXs as *S*-specific LOXs (Coffa and Brash (2004) Proc. Natl. Acad. Sci. USA 101:15579-15584).

### 7. LOX activity tests in vitro

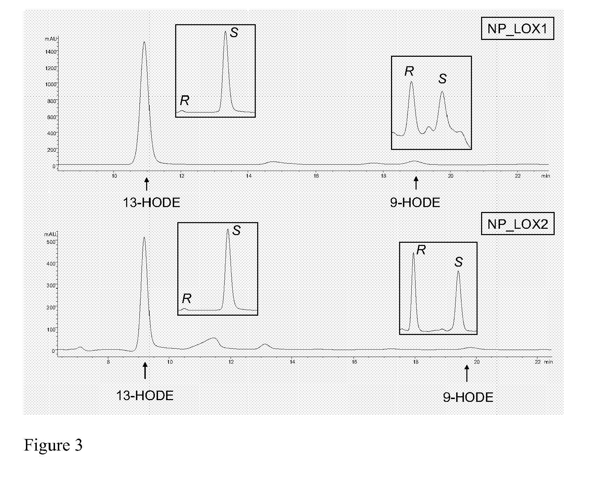

For further biochemical characterisation NpLOX1 and NpLOX2 cDNAs were expressed in *E. coli* BL21(DE3) cells and the crude cell extracts were incubated with several fatty acid substrates for activity tests. First analysis showed that both enzymes, NpLOX1 and NpLOX2, respectively, converted the C18- and C20-PUFAs to the same corresponding hydroperoxides where the oxygen was inserted at the ω-6 position. The analysis of products formed by the conversion of LA into (9*Z*,11*E*,13*S*)-13-hydro(pero)xy-octadeca-9,11-dienoic acid (13-H(P)ODE) that were detected after reduction as hydroxy fatty acids is shown in Fig. 3.

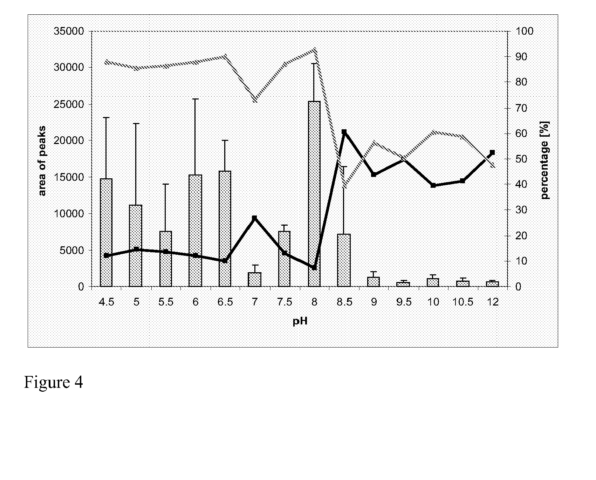

The pH-optimum of NpLOX1 was determined by summing up the integrals of all isomeric hydroxy fatty acids determined by HPLC analysis and formed by incubation with LA at pH values ranging from pH 4.5 to pH 12.0 (Bars in Fig. 4). The pH optimum was rather broad ranging from pH 4.5 to pH 8.5 with a maximum at pH 8.0. 13-H(P)ODE, (9*Z*,11*E*,13*S*,15*Z*)-13-hydro(pero)xy-9,11,15-octadecatrienoic acid (13-H(P)OTE), (6*Z*,9*Z*,11*E*,13*S*)-13-hydro(pero)xy-6,9, 11-octadecatrienoic acid (13γ-H(P)OTE) were preferred products after addition of LA, LeA and γ-linolenic acid (GLA). AA was predominantly converted to its 15S-hydroperoxide (15-H(P)ETE). Chiral phase-HPLC (CP-HPLC) analysis of the enantiomers showed that in all cases formation of the *S*-enantiomer dominated. In Table 1 the relative amounts of the hydro(pero)xides and the *S*-enantiomers are given in percentages. The highest activity and regiospecificity of NpLOX1 and NpLOX1 could be observed at pH 8.0 (92 % of 13-H(P)ODE) whereas the highest stereospecificity could be measured at pH 6.0 (Fig. 4, lines), where the relative amount of the dominating S-enantiomer of 13-H(P)ODE was about 99 % (data not shown). Furthermore, these data showed that 9-H(P)OD was mainly a product of chemical oxidation at all pH values tested, because the ratio of *S*/*R* enantiomers was almost equal. For 13-H(P)ODE the ratio of *S*/*R* enantiomers was higher than 80% at all pH values tested.

**Table 1. Fatty acid hydroperoxides formed from the reaction of NpLOX1 with different fatty acid substrates at pH 8.0. Values represent the mean of two independent experiments, standard deviation is given.**

| Substrates | Products | | | | |
|---|---|---|---|---|---|
| **Arachidonic acid** | **15-HETE** | 12-HETE | 11-HETE | 8-HETE | 5-HETE |
| Total hydroperoxides ( %) | 74 ± 10 | 1.6 ± 0.2 | 19.2 ± 14 | 4.4 ± 3.9 | n.d. |
| *S*-Enantiomer (%) | 97.4 ± 1.2 | 54.4 ± 9.1 | 77 ± 12.5 | 48.1 ± 1.9 | |
| **γ-Linolenic acid** | **13γ-HOTE** | 10γ-HOTE | 9γ-HOTE | 6γ-HOTE | |
| Total hydroperoxides ( %) | **65** ± 16 | 4.23 ± 1.2 | 6.2 ± 1.2 | 23,5 ± 15 | |
| *S*-Enantiomer (%) | 99.3 ± 0.44 | 60.5 ± 4.1 | 62.1 ± 13.9 | 63.1 ± 6.9 | |
| **α-Linolenic acid** | 16-HOTE | **13-HOTE** | 12-HOTE | 9-HOTE | |
| Total hydroperoxides ( %) | 6.57 ± 5.42 | 81.2 ± 13.1 | 5.76 ± 4.63 | 6.5 ± 3.6 | |
| *S*-Enantiomer (%) | 50 ± 3.5 | 90.2 ± 12 | 48 ± 2.2 | 51 ± 2.8 | |
| **Linoleic acid** | **13-HODE** | 9-HODE | | | |
| Total hydroperoxides ( %) | 94.3 ± 2.7 | 5.7 ± 2.7 | | | |
| *S*-Enantiomer (%) | 98.4 ± 0.2 | 60.2 ± 6.7 | | | |

| | | | | | |
|---|---|---|---|---|---|
| HETE = hydroxy arachidonic acid HODE = hydroxy octadecadienoic acid HOTE = hydroxy octadecatrienoic acid | | | | | |

The substrate preference of NpLOX1 was determined at pH 8.0 by incubating a mixture of the four different fatty acids as substrates with the enzyme extracts. The enzyme preferred LeA over LA, GLA and AA (data not shown).

### 8. LOX activity in vivo

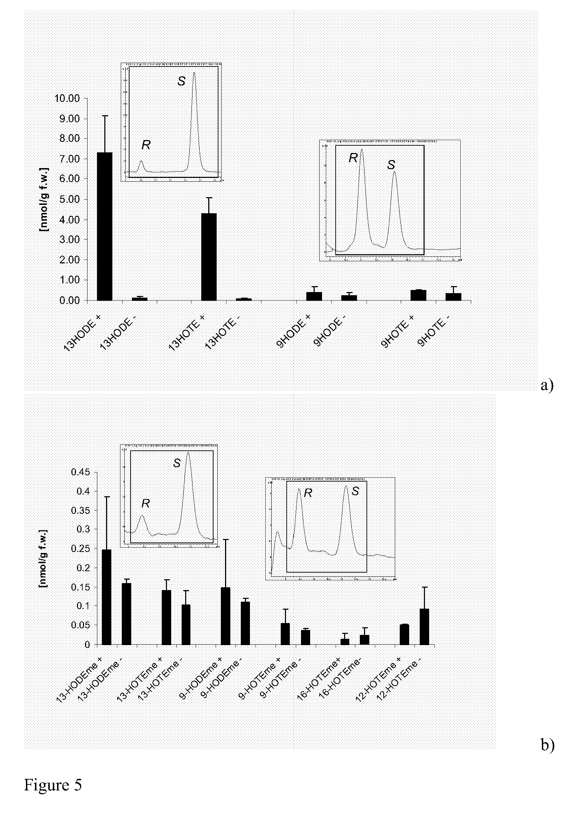

To verify the data obtained with the recombinant LOX-enzymes, the inventors aimed to analyze the LOX-pathway metabolites within the cyanobacterium *N. punctiforme* PCC73102 next. One possible role of 13-LOXs is their involvement in plants wound response. Thus the inventors compared endogenous occurring LOX pathway-products in wounded and unwounded cyanobacteria. Therefore half of the harvested cell material was wounded by sonification as described before for diatoms and mosses (Pohnert (2000) Angew. Chem. Int. Ed. Engl. 39:4352-4354; Wichard et al. (2005) Angew. Chem. Int. Ed. Engl. 44:158-161). In both, the free and esterified oxylipin fraction hydroxy- and keto dienoic fatty acids were measured. The amounts of free 13-hydroxy- and keto dienoic fatty acids were remarkably higher in samples which have been wounded whereas the amounts of 9-hydroxy and keto dienoic fatty acids did not vary a lot between wounded and unwounded cells (Fig. 5a). Chiral analysis of the identified substances showed that only the 13-HODE and 13-HOTE were products of LOX activity whereas the detected 9-HODE and 9-HOTE originated from autoxidation (Insets in Fig. 5a). For the esterified oxylipins the obtained data deviated from that of the free oxylipins. The amounts of esterified hydroxy- and keto dienoic fatty acids were close to the detectable limit, especially for the unwounded cultures, but a tendency was visible. The amounts of esterified oxylipins did only slightly increase after wounding (Fig. 5b). Chirality of the esterified 13-HODE/HOTE (detected as methyl esters: meHO(D/T)E) were as the corresponding free oxylipins products of LOX activity whereas 9-HODE/HOTE as well as 12- and 16-HOTE seemed to derive from chemical oxidation (Insets in Fig. 5b). Further oxylipins like C16- or C20-PUFA-derived hydroxides were not detected, neither in unwounded nor in wounded cyanobacteria. In addition jasmonic acid and 12-oxo phytodienoic acid were below the detection limit.

The described analyses showed that NpLOX1 and 2 were identified as linoleate 13-LOXs by expression in *Escherichia coli* (Fig. 3 & 4, Tab. 1). In principle two different regioisomers of C18 hydroperoxy PUFAs may be determined by the selectivity in the site of oxygen insertion via rearrangement of the intermediate fatty acid radical. Consequently, when hydrogen is abstracted at C-11 in either LA or LeA, molecular oxygen can be introduced at position [+2] or [-2] leading to dioxygen insertion at C-13 or C-9. Two models are currently used to explain the underlying reaction mechanism of positional specificity of LOXs (Fig. 6a) (Liavonchanka and Feussner (2006) J. Plant Physiol. 163:348-357): Based on experiments with mammalian LOXs, a space-related hypothesis was established. Here, the fatty acid substrate penetrates the active site generally with its methyl end first. Then the depth of the substrate-binding pocket determines the positional specificity of molecular oxygen insertion. According to a second hypothesis, the substrate orientation is regarded as the key step in the determination of the position of dioxygen insertion: In the case of 13-LOXs, the active site is penetrated again by the substrate using its methyl end first, whereas with 9-LOXs the substrate is forced into an inverse orientation favouring a penetration with its carboxy group first. Consequently, a radical rearrangement at either [+2] or [-2], respectively, may be facilitated in both cases by the same mechanism within the active site.

In the past site directed mutagenesis studies have mainly been carried out with mammalian LOXs. In these experiments two regions have been identified in the primary structure containing sequence determinants for the positional specificity: i) Amino acids aligning with the so-called Sloane determinants (Sloane et al. (1991), vide supra). Interestingly, they are highly conserved among plant LOXs and may determine their positional specificity (Fig. 1, s1). ii) Borngräber determinants: There is amino acid heterogeneity among plant LOXs at the position which aligns with P353 of the rabbit reticulocyte 15-LOX, but in mammalian 15- or 12-LOXs always a bulky amino acid is found at this position (Borngräber et al. (1996) J. Mol. Biol. 264:1145-1153). In addition, structural modelling of the active site of plant LOXs revealed that an arginine residue is localized in the vicinity of the putative substrate-binding pocket that seems to be a third determinant that is called Hornung determinant. It may interact with the carboxy group of the substrates when there is an inverse head to tail substrate orientation (Fig. 1, ho; Fig. 6a). In wild-type plant 13-LOXs there is in most cases a phenylalanine or a histidine located at a position which aligns with the Sloane determinants. These amino acids may shield the positive charge of the arginine and thus, the substrate may penetrate the active site with its methyl end since there is no counterpart to neutralize the charge of the carboxy group. Mutagenesis studies of the Sloane-determinants with the lipid body LOX of cucumber seedlings supported this model (Hornung et al. (1999), vide supra). In contrast, for all plant 9-LOXs, a small valine residue was identified at this highly conserved amino acid position. Structural modelling of the enzyme/substrate interaction suggests that small amino acid side chains can not mask the positively charged guanidine group of the arginine residue at the bottom of the substrate pocket. As a consequence, the guanidino group may be able to form a salt bridge with the carboxylic group of the substrate, favouring an inverse head to tail substrate orientation. However, in most mammalian LOXs analyzed so far this determinant has not been found, rendering the orientation dependent model in mammalian LOXs unlikely. Recently a fourth structural determinant that directs the stereospecific insertion of dioxygen has been identified (Coffa et al. (2005) Biochem. Biophys. Res. Commun. 338:87-92). In contrast to the amino acid side chains that determine the positional specificity, this so-called "Coffa" determinant is highly conserved between plant and mammalian LOXs (Fig. 1, cof). In summary, the space within the active site seems to be the main factor determining the positional specificity of mammalian LOXs whereas in case of plant LOXs the orientation of the substrate seems to be the major factor that determines the positional specificity.

As for all LOXs described so far the Coffa determinant is conserved in NpLOX1 and 2 identifying both enzymes as *S*-specific LOXs. Most interestingly, at the level of the other three determinants (according to Sloane, Borngräber and Hornung) the active site of both enzymes is neither similar to mammalian LOXs nor to plant LOXs (Fig. 1 and Fig. 6b).

Unlike in plant LOXs an arginine at the bottom of the substrate binding pocket is absent and in contrast to mammalian 13-LOXs the amino acid side chains at the other two positions are rather small.

The analysis of endogenous oxylipins in *Nostoc punctiforme* strain PCC73102 (Fig. 5) seems to indicate that this organism is only capable of forming oxylipins that derive directly from the action of 13-LOXs and that additional enzymes metabolizing 13-LOX-derived fatty acid hydroperoxides known from the so-called LOX pathway in higher plants are missing.

### 8. Cell-free expression of NpLOX

For the cell free expression system the Expressway^{™} Mini Cell-Free Expression System was used following the protocol of Invitrogen (Karlsruhe, Germany). The pEXP5-NT/TOPO vector containing either one of the LOX-genes (NpLOX1 or NpLOX2) was mixed with the following reagents:

| | |
|---|---|
| *E. coli slyD-* Extract | 20 µl |
| 2.5 X IVPS *E. coli* Reaction Buffer (-A.A.) | 20 µl |
| 50 mM Amino Acids (-Met) | 1.25 µl |
| 75 mM Methionine* | 1 µl |
| T7 Enzyme Mix | 1 µl |
| DNA Template | 1 µg |
| DNase/RNase-free Distilled Water To a final volume of | 50 µl |

The sample was then incubated at 30 C for 30 min on a shaker (300 rpm). After incubation 50 µl of the prepared Feed Buffer was added and the reaction was incubated for another 3-6 hours at 30-37 C. The sample was then stored at -20 °C prior to further use.

### 9. Purifying the recombinant soluble His-tagged fusion protein

Both LOXs were fused to an N-terminal 6xHis-tag, a short poly-histidine peptide whose imidazole rings chelate divalent metal ions like Co²⁺, Zn²⁺, Ni²⁺ or Fe²⁺. This metal chelating property allows the purification of NpLOX1 and NpLOX2 by *immobilized metal ion affinity chromatography* (IMAC).
The purification of His-LOX fusion proteins was performed with TALON^{™} Resin (ClonTech, U.S.), that uses cobalt ions for purifying recombinant polyhistidine-tagged proteins. If proteins were expressed *in vivo* in *E. coli,* the samples were prepared as follows. The cell culture was harvested via centrifugation (4000 rpm, 4°C, 20 min) and the pellet resuspended in 2 ml lysis buffer (50mM TrisHCl pH 8, 300mM NaCl, 10% glycerol, 0.1% Tween20) containing 0.75 mg/ml lysozyme. The samples were incubated for 30 min at RT and after that sonicated for 2x 20 sec with 20 sec pause in between. The crude extract was pelleted via centrifugation (4000 rpm, 4 °C, 10 min) and the supernatant containing the soluble protein transferred to a clean tube. Before applying the sample to the resin, EDTA was removed by using a gel filtration column (PD-10, GE Healthcare) with the Equilibration/Wash Buffer.
The TALON was equilibrated 3 x with lysis buffer and than mixed in a 50 ml tube with the supernatant containing the protein. The mixture was shaken at 4 C over night. Next day, the tubes were put on ice until the column material was sedimented. The supernatant was removed carefully until 5 ml of buffer were left. The column material was transferred into an empty column and washed 3 x with 5 ml of washing buffer (50mM TrisHCl, 300mM NaCl, 0.1% Tween 20, 10 mM Imidazol, pH 8.0). The flow-through was collected in 15 ml tubes. After washing the column the protein was eluated in several steps (3 x 1 ml and 1 x 10 ml) with elution buffer (50mM TrisHCl pH 8, 300mM NaCl, 0,1 % Tween 20, 120mM Imidazol) and collected in different tubes.
The collected samples were checked SDS PAGE analysis to determine the fraction(s) which contained the majority of purified protein. After purification the fused tag can be removed of the protein with a specific protease. The pEXPS-NT/TOPO vector contains a Tobacco Etch Virus (TEV) recognition site to remove the N-terminal His-tag from the fusion protein with the TEV protease.

### 10. Purifying the recombinant soluble pMAL-tagged Fusion Protein

In addition to the His-tagged fusion proteins, the LOXs could also be expressed using the pMAL-c2x-vector (NewEnglandBiolabs, U.S.). Instead of an N-terminal His-tag the LOX gene is fused to the malE gene which encodes maltose-binding protein (MBP). The expression of the fused genes results in the synthesis of an MBP fusion protein.
For the purification of MBP-LOX fusion protein affinity chromatography was performed with amylase resins as column matrix following the user manual protocol. The amylose resins were poured into an empty column washed 8 x with column buffer (50mM TrisHCl pH 8.0, 300 mM NaCl) and then loaded with diluted crude extract. After several washing steps with column buffer the fusion protein was eluted with buffer containing 10mM maltose.
The collected fractions were tested with SDS-PAGE-analysis to determine the fraction with majority of protein.
After purification the fusion protein can be cleaved with a specific protease recognizing the Factor Xa cleavage site. Protease and recombinant protein can then further be purified with ion exchange chromatography.

### DESCRIPTION OF THE FIGURES

### Fig. 1

Partial amino acid sequence alignment of the active site residues of NpLOX1 and NpLOX2 in comparison with 13-LOXAtLOX3 (Acc. no. AAF79461) from *Arabidopsis thaliana,* with 13-LOX PpLOX1 (Acc. no. CAE47464) from *Physcomitrella patens,* with 13-LOX PaLOX1 (Acc. no. AAL85880) from *Pseudomonas aeruginosa* and with 8R-LOX PhLOX1 (Acc. no. AAC47283) from the coral *Plexaura homomalla.* For alignment, the MultAlign program was used (Corpet (1988) Nucl. Acids Res. 16: 10881-10890). The following conserved amino acids are *framed:* the residues involved in iron ligation (three histidins, an asparagine and an isoleucin; *li*), and the three determinants for substrate and regiospecificity (*bo,* according to Borngräber et al. (1996), vide supra, *cof,* according to Coffa and Brash (2004), vide supra, *sl,* according to Sloane et al. (1991), vide supra, *ho,* according to Hornung et al. (1999), vide supra). The numbering of amino acid refers to the amino acid sequence of NpLOX1.

### Fig. 2

Expression analysis of NpLOX2 in the bacterial strains BL21, BL21*, Rosetta (Ros), HMS and RP transformed with pEX5-NT-NpLOX2. Both the supernatant (SN) and the pellet (P) after lysis of the cells were separated on a 10% SDS gel and then stained with Coomassie. Both extracts from cells stressed with 6% ethanol and from unstressed cells were separated.

### Fig. 3

Analysis of products formed by NpLOX1 and NpLOX2, respectively, with LA at pH 8.0 (here in reduced form for SP-HPLC analysis). Insets show analysis of *R* and *S* enantiomer ratio by CP-HPLC analysis.

### Fig. 4

Enzyme activity and regiospecificity of H(P)ODE production of NpLOX1 depending on different pH-values. The sum of the peak areas determined by HPLC for 9- and 13-H(P)ODE is shown in grey bars as a measure for LOX activity. The regiospecificity is presented by the percentages of 9-H(P)ODE (black curve) and 13-H(P)ODE (grey curve), respectively. Each value represents the average of two independent experiments.

### Fig. 5

HPLC-analysis of endogenous a) non esterified (HODE) and b) esterified HODEs (HODEme) of *N. punctiforme* PCC73102. The bars represent the values of detected hydroxy fatty acids from unwounded (-) or wounded (+) cyanobacteria. Each value represents an average of two independent experiments, standard deviation is given. The insets show the CP-HPLC analyses for 13-HODE and 13-HODEme, respectively. 13-HOTE and 13-HOTEme represent enzymatically produced hydroxy fatty acids whereas 9-HODE, 9-HODEme, 12-HOTEme and 16-HOTEme represent the hydroxy fatty acids produced by chemical oxidation.

### Fig. 6

Comparison of the two different models explaining the positional specificity of LOXs a) space-related theory: substrate penetrates the active site of LOXs always with its methyl end, depending on available space in the active site; and b) orientation-dependent theory: straight or inverse-substrate orientation depending on interacting amino acid residues in the acitve site. c) Model showing the amino acid residues corresponding to the active side residues explained in a) and b) within the active site of NpLOX1 and NpLOX2, respectively.

## Claims

1. Method of producing polyunsaturated fatty acid hydroperoxides from polyunsaturated fatty acids by use of a protein having the enzymatic activity of a prokaryotic 13-lipoxygenase.

2. The method according to claim 1, wherein the 13-lipoxygenase originates from a bacterium, preferably from a cyanobacterium.

3. The method according to claim 1 or 2, wherein the 13-lipoxygenase originates from a member of the *Nostocaceae* family, preferably from *Nostoc punctiforme.*

4. The method according to any one of the preceding claims, wherein the protein is encoded by a nucleic acid sequence selected from the group consisting of:
a) nucleic acid sequences comprising a nucleotide sequence encoding a protein with the amino acid sequence depicted in SEQ ID NOs: 1 or 2, or comprising a fragment of said nucleotide sequence, wherein said fragment is sufficient to code for a protein having the enzymatic activity of a 13-lipoxygenase,
b) nucleic acid sequences comprising the nucleotide sequence depicted in SEQ ID NOs: 3 or 4, or comprising a fragment of said nucleotide sequence, wherein said fragment is sufficient to code for a protein having the enzymatic activity of a 13-lipoxygenase,
c) nucleic acid sequences comprising a nucleotide sequence which hybridizes to a complementary strand of the nucleotide sequence from a) or b) under stringent conditions, or comprising a fragment of said nucleotide sequence, wherein the fragment is sufficient to code for a protein having the enzymatic activity of a 13-lipoxygenase,
d) nucleic acid sequences comprising a nucleotide sequence which shows at least 60 % identity to the nucleotide sequence from a), b) or c), or comprising fragments of said nucleotide sequence, wherein the fragment is sufficient to code for a protein having the enzymatic activity of a 13-lipoxygenase.

5. The method according to any one of the preceding claims, further comprising
- recovering the produced polyunsaturated fatty acid hydroperoxides and/or
- using the produced polyunsaturated fatty acid hydroperoxides for the production of flavor ingredients and/or fragrance ingredients and/or
- using the produced polyunsaturated fatty acid hydroperoxides as pesticidal or fungicidal compounds and/or
- using the produced polyunsaturated fatty acid hydroperoxides for the production of pesticidal or fungicidal compounds.

6. The method according to claim 5,
wherein the flavor ingredients and/or fragrance ingredients are produced from the fatty acid hydroperoxides by cleavage to aldehydes and optionally reduction to the corresponding alcohols.

7. The method according to claim 6,
wherein the fatty acids hydroperoxides are cleaved by a hydroperoxide lyase.

8. The method according to any one of the preceding claims, wherein the production of the polyunsaturated fatty acid hydroperoxides occurs *in vitro.*

9. The method according to claim 8, wherein a purified 13-lipoxygenase protein is used for producing the polyunsaturated fatty acid hydroperoxides.

10. The method according to any one of claims 1 to 8, wherein the production of the polyunsaturated fatty acid hydroperoxides occurs in a transgenic organism, transgenic cell or in an extract from said organism or cell.

11. The method according to claim 10, wherein the organism or cell is a bacterium, a fungus, a yeast, a plant or plant cell.

12. The method according to any one of claims 10 or 11, wherein the organism is is a Gram negative bacterium such as *E. coli,* a Gram positive bacterium such *B. subtilis,* a fungus such as *A. niger, A. nidulans, N. crassa;* a yeast such as *S. cerevisiae, K. lactis, H. polymorpha, P. pastoris, Y lipolytica, etc;* actinomycetes such as *Streptomyces* sp.

13. The method according to any one of claims 10 to 12, wherein the organism has been transformed with a recombinant nucleic acid molecule comprising a nucleic acid sequence as defined in claim 4.

14. Isolated nucleic acid molecule comprising a nucleic acid sequence encoding a protein having the enzymatic activity of a 13-lipoxygenase, said nucleic acid sequence being selected from the group consisting of:
a) nucleic acid sequences comprising a nucleotide sequence encoding a protein with the amino acid sequence depicted in SEQ ID NOs: 1 or 2, or comprising a fragment of said nucleotide sequence, wherein said fragment is sufficient to code for a protein having the enzymatic activity of a 13-lipoxygenase,
b) nucleic acid sequences comprising the nucleotide sequence depicted in SEQ ID NOs: 3 or 4, or comprising a fragment of said nucleotide sequence, wherein said fragment is sufficient to code for a protein having the enzymatic activity of a 13-lipoxygenase,
c) nucleic acid sequences comprising a nucleotide sequence which hybridizes to a complementary strand of the nucleotide sequence from a) or b) under stringent conditions, or comprising a fragment of said nucleotide sequence, wherein the fragment is sufficient to code for a protein having the enzymatic activity of a 13-lipoxygenase,
d) nucleic acid sequences comprising a nucleotide sequence which shows at least 60 % identity to the nucleotide sequence from a), b) or c), or comprising fragments of said nucleotide sequence, wherein the fragment is sufficient to code for a protein having the enzymatic activity of a 13-lipoxygenase.

15. The nucleic acid sequence according to claim 14, originating from a bacterium, preferably from a cyanobacterium.

16. The nucleic acid sequence according to any of claims 14 or 15, originating from a member of the *Nostocaceae* family, preferably from *Nostoc punctiforme.*

17. Recombinant nucleic acid molecule comprising the following elements:
- regulatory sequences of a promoter which is active in a target cell,
- operatively linked thereto a nucleic acid sequence according to any one of claims 14 to 16, and
- optionally operatively linked thereto regulatory sequences which can serve as transcription, termination and/or polyadenylation signals in the target cell.

18. The recombinant nucleic acid molecule according to claim 17, further comprising a nucleic acid sequence coding for an affinity tag.

19. The recombinant nucleic acid molecule according to claim 18, wherein the affinity tag is selected from the group consisting of maltose binding domain, cellulose binding domain, protein A, histidine and glutathione-S-transferase.

20. The recombinant nucleic acid molecule according to any of claims 17 to 19,
wherein the target cell is a bacterial or plant cell.

21. Recombinant protein having the enzymatic activity of a 13-lipoxygenase and an amino acid sequence which is encoded by a nucleic acid sequence according to any one of claims 14 to 16 or which is produced by the expression of a recombinant nucleic acid molecule according to any one of claims 17 to 20.

22. Method of producing the recombinant protein according to claim 21 in a transgenic organism or transgenic cell, wherein the recombinant nucleic acid molecule according to any one of claims 17 to 20 is transferred to and expressed in said organism or cell.

23. The method according to claim 22, wherein the organism or cell is a bacterium, a fungus, a yeast, a plant or plant cell.

24. The method according to claim 22 or 23, further comprising the step of recovering the recombinant protein from the transgenic organism, transgenic cell or the media.

25. The method of claim 24, wherein the protein is recovered by means of an affinity tag.

26. The method of claim 25, further comprising the immobilization of the recovered protein on a matrix.

27. The method of claim 26, wherein the matrix is amylose or cellulose.

28. Method of generating transgenic plants, transgenic plant cells, transgenic yeast cells, transgenic fungi or transgenic bacterial cells having an increased content of polyunsaturated fatty acid hydroperoxides compared to wild-type plants or wild-type cells or wild-type fungi due to an increased enzymatic activity of 13-lipoxygenase compared to wild-type plants or wild-type cells or wild-type fungi, comprising the following steps:
a) producing a recombinant nucleic acid molecule according to any one of claims 17 to 20;
b) transferring the nucleic acid molecule from a) to plant cells or yeast cells or bacterial cells or fungi.

29. Method of generating transgenic plants, transgenic plant cells, transgenic yeast cells, transgenic fungi or transgenic bacterial cells having an increased content of flavor ingredients and/or fragrance ingredients compared to wild-type plants or wild-type cells or wild-type fungi, comprising the following steps:
a) producing a recombinant nucleic acid molecule according to any one of claims 17 to 20;
b) producing a recombinant nucleic acid molecule comprising the following elements:
- regulatory sequences of a promoter which is active in a target cell,
- operatively linked thereto a nucleic acid sequence encoding a hydroperoxide lyase, and
- optionally operatively linked thereto regulatory sequences which can serve as transcription, termination and/or polyadenylation signals in the target cell;
c) transferring the nucleic acid molecules from a) and b) to plant cells or yeast cells or bacterial cells or fungi.

30. Transgenic plant cell or yeast cell or bacterial cell or fungus containing a recombinant nucleic acid molecule according to any one of claims 17 to 20 or generated by a method according to claim 28 or 29 and which exhibits a new or altered expression of the recombinant protein according to claim 21 compared to corresponding wild type cells.

31. A method of producing 13-lipoxygenase-derived hydroperoxides in transgenic plants or transgenic plant cells or transgenic yeast cells or transgenic bacterial cells or transgenic fungi, comprising the following steps:
a) transferring a nucleic acid sequence or a nucleic acid molecule according to any one of claims 14 to 20 to plants or plant cells or yeast cells or bacterial cells or fungi;
b) producing 13-lipoxygenase-derived hydroperoxides by expression of the nucleic acid sequence according to any one of claims 14 to 20 in the transgenic cells, optionally with adding α-linolenic acid, linoleic acid and/or γ-linolenic acid to said cells; and
c) obtaining the produced 13-lipoxygenase-derived hydroperoxides from these cells, plants or the media wherein the cells or plants are cultivated during the production step.

32. A method of producing 13-lipoxygenase-derived hydroperoxides *in vitro* comprising:
- providing a protein according to claim 21 or producing said protein by a method according to any one of claims 22 to 27;
- contacting said protein with α-linolenic acid, linoleic acid and/or γ-linolenic acid or their natural precursors under suitable reaction conditions; and
- recovering the produced 13-lipoxygenase-derived hydroperoxides.

33. A method of producing flavor ingredients and/or fragrance ingredients *in vitro* comprising:
- providing a protein according to claim 21 or producing said protein by a method according to any one of claims 22 to 27 and providing a protein having the enzymatic activity of a hydroperoxide lyase;
- contacting said proteins with α-linolenic acid, linoleic acid and/or γ-linolenic acid or their natural precursors under suitable reaction conditions; and
- recovering the produced aldehydes as flavor ingredients and/or fragrance ingredients.

34. Method according to claim 33, further comprising reducing the aldehydes to the corresponding alcohols.

35. Use of the hydroperoxides produced by the process according to any one of claims 1 to 13 and 31 or 32 for the synthesis of flavor ingredients and/or fragrance ingredients.

36. Use of the hydroperoxides produced by the process according to any one of claims 1 to 13 and 31 or 32 as pesticidal or fungicidal compounds in controlling plant diseases.
